(19) Europäisches Patentamt
European Patent Office
Office européen des brevets

(11) **EP 4 462 122 A1**

(12) **EUROPEAN PATENT APPLICATION**

(43) Date of publication:
**13.11.2024 Bulletin 2024/46**

(21) Application number: **23172075.6**

(22) Date of filing: **08.05.2023**

(51) International Patent Classification (IPC):
**G01N 33/543** (2006.01)   **B01L 3/00** (2006.01)
**G01N 35/00** (2006.01)   **B32B 37/00** (2006.01)

(52) Cooperative Patent Classification (CPC):
**G01N 35/00029**; B32B 37/025; G01N 2035/00108;
**G01N 2035/00168**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Roche Diagnostics GmbH
68305 Mannheim (DE)**

(72) Inventors:
• **BLUM, Martin**
  **68305 Mannheim (DE)**
• **ISGOEREN, Yilmaz**
  **68305 Mannheim (DE)**
• **MEYER DOS SANTOS, Sascha**
  **68305 Mannheim (DE)**

(74) Representative: **Altmann Stößel Dick
Patentanwälte PartG mbB
Theodor-Heuss-Anlage 2
68165 Mannheim (DE)**

(54) **METHOD OF MANUFACTURING A PLURALITY OF ANALYTICAL TEST ELEMENTS**

(57)     A method and a manufacturing device (110) for manufacturing a plurality of analytical, specifically diagnostic, test elements (112) comprising at least one core strip (114) and at least one support foil strip (116) is disclosed. The method comprises:

i. providing at least one core strip intermediate product (120), the core strip intermediate product (120) comprising at least one carrier foil (122) and at least one analytical element (124) attached to the at least one carrier foil (122), the carrier foil (122) and the attached analytical element (124), in the carrier intermediate product (120), being cut such that a plurality of core strips (114) of the analytical test elements (112) is defined, the core strips (114) being placed adjacent to each other;

ii. applying transfer foils (128) on a front side (130) and on a reverse side (132) of the core strip intermediate product (120), wherein the transfer foil (128) applied to the front side (130) and the transfer foil (128) applied to the reverse side (132) engage with the core strips (114) in an alternating fashion, the transfer foil (128) on the front side (130) and the core strips (114) engaging with the transfer foil (128) on the front side (130) forming a first transfer intermediate product (134), the transfer foil (128) on the reverse side (132) and the core strips (114) engaging with the transfer foil (128) on the reverse side (132) forming a second transfer intermediate product (136);

iii. separating the first (134) and second transfer intermediate products (136); and

iv. transferring the core strips (114) onto at least one support foil (142), wherein the core strips (114), on the support foil (142), are spaced apart from each other by at least one width of one core strip (114).

Fig. 1

## Description

Technical Field

The present invention refers to a method of manufacturing a plurality of analytical, specifically diagnostic, test elements and to a manufacturing device for manufacturing the plurality of analytical, specifically diagnostic, test elements. The method and device, as an example, may be used for manufacturing a plurality of analytical test elements for lateral flow assays used in the field of medical diagnostics for detecting one or more properties of a sample of a bodily fluid, e.g. a presence and/or a concentration of at least one analyte in the sample of the bodily fluid. Other fields of application of the present invention, however, are also feasible.

Background art

Analytical test elements comprising one or more test chemicals, which, in presence of an analyte to be detected, are capable of performing one or more detectable detection reactions, such as optically detectable detection reactions, are frequently used in analytical applications. For example, in the field of medical diagnostics, such in point of care applications and/or in laboratories, samples of a bodily fluid, such as saliva, blood, interstitial fluid, urine, salvia, sweat, serum or other types of bodily fluids, have to be analyzed, for example in order to detect a presence and/or a concentration of an analyte in the sample of the bodily fluid. Examples of analytes to be detected are antigenic proteins of viruses, such as antigenic proteins of the SARS-CoV-2 coronavirus, and other types of analytes, such as glucose, triglycerides, lactate, cholesterol or other types of analytes typically present in these bodily fluids. Generally, methods and systems of producing these analytical test elements are known which, for example, use a reel-to-reel process for manufacturing core strips, wherein the core strips may subsequently be introduced in disposable, such as plastic housing, which have a bigger size compared with the core strips. For example, US 2022/0305766 A1 discloses a method of producing a plurality of analytical test strips using a reel-to-reel process, comprising: providing at least one continuous first layer web, having disposed on a first side at least one first electrode layer, the first layer web having a first layer edge; continuously disposing at least one continuous spacer layer web onto the first side of the first layer web, wherein the spacer layer web has a spacer layer edge, wherein the disposing is position-controlled in a master-slave fashion by using a position of the first layer edge as a master position and a position of the spacer layer edge as a slave position; and continuously disposing at least one continuous second layer web onto the spacer layer web, the second layer web having disposed on a first side at least one second electrode layer, wherein the second layer web has a second layer edge.

US 2002/0076828 A1 describes a chromatography immunoassay device which can be used more easily, is protected from moisture and/or oxygen and can be produced at a low cost. The chromatography immunoassay device is one in which one or more chromatography strips are stuck on a substrate made of a plate, each of the chromatography strips is sealed by closely adhering a substrate portion surrounding each chromatography strip to a seal film located on the chromatography strip, and the seal film and/or substrate possesses a film containing a dehumidifying agent and/or a film containing an oxygen absorbing agent.

US 6,207,000 B1 discloses process for the production of analytical devices. Analytical devices include analytical test elements with a capillary active zone for examining liquid samples. In the process a carrier layer is prepared, a spacer layer is laminated onto the carrier layer, a contour is punched, cut or stamped through the spacer layer laminated onto the carrier layer which determines the shape of the capillary-active zone, those parts of the spacer layer which are not required to form the capillary-active zone are removed from the carrier layer and a cover layer is applied to the spacer layer to result in a capillary-active zone. The process is preferably suitable for manufacturing analytical devices from tape material.

US 2008/0289749 A1 describes a method for manufacturing a diagnostic test strip. The method includes the acts of providing an application sheet having a plurality of adhesive dots thereon, providing a first substrate layer having at least one feature located thereon, and providing a second substrate layer. The method further includes the acts of transferring at least one of the plurality of adhesive dots located on the application sheet to the first substrate layer, aligning the first substrate layer with the second substrate layer, and attaching the first substrate layer and the second substrate layer using the transferred adhesive dots, wherein the attaching of the first and second substrate layers is performed without any additional alignment.

EP 2 907 573 A1 discloses a process for the production of at least one analytical device. The analytical device has at least one capillary element. The process comprises the following steps: a) providing at least one carrier layer; b) providing at least one spacer layer; c) applying the spacer layer on top of the carrier layer; d) providing at least one cover layer; e) applying the cover layer on top of the spacer layer. The process further comprises at least one cutting step, wherein at least one capillary channel of the capillary element is cut out from the spacer layer. The cutting step is performed by using at least two cutting tools, the cutting tools complementing one another to form a contour of the capillary channel.

Despite the advantages achieved by known method and devices, several technical challenges remain. Specifically, introducing small core strips, e.g. of sizes smaller than 10 mm in at least one dimension, into disposables or housings exceeding the size of the core strips may generally be envisaged in order to improve the handling of the analytical test element by the user. However, as outlined above, core strips are generally produced using continuous processes, such as a reel-to-reel process. In subsequent manufacturing steps, the produced core strips may be transferred or introduced into the disposables by packaging and labelling, e.g. by using pick-and-place methods. These subsequent manufacturing steps are generally time- and cost-intensive and required additional hardware equipment. Thus, in general, known manufacturing methods and devices are disadvantageously with respect to manufacturing time and costs.

Problem to be solved

It is therefore desirable to provide methods and devices that at least partially address the above-mentioned technical challenges. Specifically, a method of manufacturing and a manufacturing device shall be proposed which allow manufacturing a plurality of analytical test elements at low cost and/or high throughput and, preferably, with a reduction in equipment requirements.

Summary

This problem is addressed by a method of manufacturing a plurality of analytical test elements and by a manufacturing device with the features of the independent claims. Advantageous embodiments which might be realized in an isolated fashion or in any arbitrary combinations are listed in the dependent claims as well as throughout the specification.
As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.
Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.
Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.
In a first aspect of the present invention, a method of manufacturing a plurality of analytical, specifically diagnostic, test elements comprising at least one core strip and at least one support foil strip, is disclosed. In the following, the method may also be referred to as "manufacturing method".
The method comprises the following steps, which, as an example, may be performed in the given order. It shall be noted, however, that a different order is also possible. Further, it is possible to perform one or more or even all of the method steps once or repeatedly. Further, it is possible to perform two or more of the method steps simultaneously or in a timely overlapping fashion. The method may comprise additional method steps, which are not listed.
The method comprises:

i. providing at least one core strip intermediate product, the core strip intermediate product comprising at least one carrier foil and at least one analytical element attached to the at least one carrier foil, the carrier foil and the attached analytical element, in the carrier intermediate product, being cut such that a plurality of core strips of the analytical test elements is defined, the core strips being placed adjacent to each other;
ii. applying transfer foils on a front side and on a reverse side of the core strip intermediate product, wherein the transfer foil applied to the front side and the transfer foil applied to the reverse side engage with the core strips in an alternating fashion, the transfer foil on the front side and the core strips engaging with the transfer foil on the front side forming a first transfer intermediate product, the transfer foil on the reverse side and the core strips engaging with the transfer foil on the reverse side forming a second transfer intermediate product;
iii. separating the first and second transfer intermediate products; and
iv. transferring the core strips onto at least one support foil, wherein the core strips, on the support foil, are spaced apart from each other by at least one width of one core strip.

The term "manufacturing" as used herein is a broad term and is to be given its ordinary and customary meaning to a person of ordinary skill in the art and is not to be limited to a special or customized meaning. The term specifically may refer, without limitation, to a process of producing at least one final product from one or more base products. Specifically, the manufacturing process may comprise processing and/or transforming one or more base products into the final product. The final product of the manufacturing method may comprise the plurality of analytical test elements. The manufacturing of the plurality of analytical test elements may comprise processing at least one element and/or component in such as manner as to retrieve the plurality of analytical test elements.

The term "analytical test element" as used herein is a broad term and is to be given its ordinary and customary meaning to a person of ordinary skill in the art and is not to be limited to a special or customized meaning. The term specifically may refer, without limitation, to an element or device configured for determining a presence and/or a concentration of at least one analyte in a liquid sample. The analytical test element may specifically be an analytical test strip, such as by having a strip-shaped form, as will be outlined in further detail below. The analytical test strip may comprise at least one component, which changes at least one detectable property, specifically at least one of an optically detectable property and an electrochemically detectable property, when the analyte is present in the liquid sample and/or according to a concentration of the analyte in the liquid sample. The analytical test element may comprise at least one lateral flow assay. The analytical test element may be configured for an *in-vitro* measurement. The analytical test element may be an electrochemical test strip. The analytical test element may comprise one or more electrodes, such as an electrode structure configured for electrically connecting the analytical test element to a meter, e.g. a blood glucose meter. As an example, the analytical test element may be a diagnostic test element. The analytical test element may be configured for determining a presence and/or a concentration of at least one analyte in a bodily fluid, specifically in a sample of a bodily fluid. The analyte to be detected may be or may comprise at least one of an antigenic protein of a virus, such as an antigenic protein of SARS-CoV-2 coronavirus, Influenza virus A, Influenza virus B, HIV or other viruses, and an analyte involved in metabolism, such as glucose, triglycerides, lactate, cholesterol or any other analyte. The bodily fluid may be or may comprise saliva, nasal swab, throat swab, blood, interstitial fluid, urine, salvia, sweat, serum or any other type of bodily fluid. As another example, the analytical test element may be a test element used in the field of analytical chemistry, such as a test element involving one or more color-changing indicators. By way of example, such analytical test elements may comprise test elements for determining a presence and/or a concentration of at least one analyte in water, such as analytical test element used for testing water quality of fish tanks, specifically determining a presence and/or a concentration of one or more of pH value, carbonate, nitrate, nitrite, total hardness, total alkalinity and/or other chemical elements or compounds.

The term "strip" or "strip-shaped" as used herein is a broad term and is to be given its ordinary and customary meaning to a person of ordinary skill in the art and is not to be limited to a special or customized meaning. The term specifically may refer, without limitation, to an element having an elongated shape and a thickness, wherein an extension of the element in a lateral dimension exceeds the thickness of the element, such as by at least a factor of 2, preferably by at least a factor of 5, more preferably by at least a factor of 10 and most preferably by at least a factor of 20 or even at least a factor of 30.

The term "core strip" as used herein is a broad term and is to be given its ordinary and customary meaning to a person of ordinary skill in the art and is not to be limited to a special or customized meaning. The term specifically may refer, without limitation, to at least one functional element of the analytical test element. Specifically, the core strip may comprise one or more elements being configured for providing alone or in combination with each other the analytical functionality of the analytical test element, such as one or more elements being configured for determining the presence and/or the concentration of the at least one analyte in the liquid sample. The core strip may be the lateral flow assay of the analytical test element. The core strip may comprise, as will be outlined in further detail below, at least one carrier material, such as at least one strip of the carrier foil, and, disposed thereon or therein, the at least one analytical element. The core strip may be a strip-shaped element, as defined above. The core strip may be at least partially enclosed by one or more other elements of the analytical test element, e.g. by one or more other elements providing support and/or sealing for the core strip.

The term "foil" as used herein is a broad term and is to be given its ordinary and customary meaning to a person of ordinary skill in the art and is not to be limited to a special or customized meaning. The term specifically may refer, without limitation, to a thin sheet, plate, strip and/or web of material. The foil may be referred as thin if a thickness of the foil does not exceed 1 cm, specifically 5 mm, more specifically 1 mm. The foil may be provided in continuous form, e.g. on a roll or reel. The foil may comprise at least one flexible or deformable material, such as at least one flexible or deformable plastic foil. The foil may comprise at least one plastic material, such as polyester, polyethylene terephthalate, polyethylene or the like. Additionally or alternatively, the foil may comprise at least one metal, such as aluminum, copper, tin, gold or the like.

The term "support foil" as used herein is a broad term and is to be given its ordinary and customary meaning to a person of ordinary skill in the art and is not to be limited to a special or customized meaning. The term specifically may refer, without limitation, to at least one foil providing a base for at least one other element. The support foil may provide a base for the core strip, specifically for the plurality of core strips from at least one of the transfer intermediate products. The support foil may be configured for receiving the core strips. The support foil may be part of the manufactured plurality of analytical test elements. The support foil may be configured for at least partially enclosing the core strip, e.g. by providing, alone or in combination with one or more further elements, sealing for the manufactured plurality of analytical test elements.

The term "support foil strip" as used herein is a broad term and is to be given its ordinary and customary meaning to a person of ordinary skill in the art and is not to be limited to a special or customized meaning. The term specifically may refer, without limitation, to a single strip of the support foil. The support foil strip may be part of the support foil, e.g. by being cut out of the support foil and/or by being punched out of the support foil. Such a cutting or punching step may be part of the manufacturing method. Alternatively, the support foils strips may not be separated during the manufacturing method. For example, the manufactured plurality of analytical test element may comprise a plurality of support foil strips, wherein the plurality of support foil strips may be connected with each other, specifically with neighboring support foil strips. The plurality of support foil strips may be configured for being separated from each other, e.g. upon usage of the analytical test element at a user on site. For example, the manufactured plurality of analytical test elements may be connected with each other to form at least one sheet or web, wherein the plurality of analytical test elements may be connected via their respective support foil strip.

As outlined above, step i. of the method comprises providing the at least one core strip intermediate product. The term "providing" as used herein is a broad term and is to be given its ordinary and customary meaning to a person of ordinary skill in the art and is not to be limited to a special or customized meaning. The term specifically may refer, without limitation, to a process of supplying and/or making available. Specifically, the providing of the core strip intermediate product in step i. may comprise making the core strip intermediate product available for further processing. The providing of the core strip intermediate product may comprise supplying the core strip intermediate product to a manufacturing device, as will be described in further detail below, and/or producing the core strip intermediate product from one or more base products.

The term "intermediate product" as used herein is a broad term and is to be given its ordinary and customary meaning to a person of ordinary skill in the art and is not to be limited to a special or customized meaning. The term specifically may refer, without limitation, to an element being manufactured from simpler base products and intended for further processing of more complex products. Specifically, the intermediate products may be used for further processing to manufacture the plurality of analytical test elements. The intermediate product may at least partially form part of the manufactured plurality of analytical test elements. Alternatively, in other examples, the intermediate product may not be part of the manufactured plurality of analytical test elements but may be used in one or more processing steps to manufacture the plurality of analytical test elements.

The term "core strip intermediate product" as used herein is a broad term and is to be given its ordinary and customary meaning to a person of ordinary skill in the art and is not to be limited to a special or customized meaning. The term specifically may refer, without limitation, to an intermediate product comprising one or more core strips. Specifically, the core strip intermediate product may comprise the plurality of core strips. The core strip intermediate product may be provided as a sheet or web, specifically as a continuous sheet or web. For example, the core strip intermediate product may be provided in continuous form on a roll or reel. A width of the core strip intermediate product may exceed a width of the core strips. The width of the core strip intermediate product may exceed the width of the core strips by a factor of 1.5, specifically by a factor or 2, more specifically by a factor of 3. For example, the core strips may have a width of 70 mm. The core strip intermediate product may have a width of 210 mm. The core strip intermediate product may at least partially form part of the manufactured plurality of analytical test elements. Specifically, the core strips of the core strip intermediate product may form part of the manufactured plurality of analytical test elements.

The core strip intermediate product may be the base product for performing the manufacturing method. Alternatively, the manufacturing method may comprise manufacturing the core strip intermediate product from one or more simpler base products. Step i. may comprise attaching the at least one analytical element to the at least one carrier foil, and further cutting the carrier foil and the attached analytical element such that the plurality of core strips is formed. Specifically, the core strip intermediate product may be manufactured from the carrier foil by attaching the analytical element to the carrier foil and cutting the carrier foil and the attached analytical element.

The core strip intermediate product may have a longitudinal direction of extension, specifically a longitudinal direction of extension of a carrier foil web. The core strip intermediate product may be cut such that the plurality of core strips of the analytical test elements is oriented essentially perpendicular to the longitudinal direction of extension. The core strip intermediate product may be cut such that the plurality of core strips of the analytical test elements is defined by a plurality of parallel cutting lines.

As outlined above, the core strip intermediate product comprises the at least one carrier foil and the at least one analytical element attached to the at least one carrier foil. The term "carrier foil" as used herein is a broad term and is to be given its ordinary and customary meaning to a person of ordinary skill in the art and is not to be limited to a special or customized meaning. The term specifically may refer, without limitation, to a foil providing at least one substrate for carrying the analytical element. Specifically, the carrier foil may have disposed thereon or therein the analytical element of the core strip. The carrier foil may specifically be made from at least one plastic material, such as polyester, polyethylene terephthalate, polyethylene or the like, in particular from polyethylene terephthalate.

The term "analytical element" as used herein is a broad term and is to be given its ordinary and customary meaning to a person of ordinary skill in the art and is not to be limited to a special or customized meaning. The term specifically may refer, without limitation, to at least one element configured for providing alone or in combination with each other the analytical functionality of the analytical test element, such as one or more elements being configured for determining the presence and/or the concentration of the at least one analyte in the liquid sample. The analytical element may specifically be an element of the core strip. The analytical element may be selected from the group consisting of: a spreading fleece for receiving at least one liquid sample, specifically at least one liquid sample of a bodily fluid and/or at least one liquid sample obtained from a bodily fluid; at least one detection material, the detection material being configured for changing the at least one measurable property in the presence of at least one analyte to be detected, specifically at least one of an optically detectable property and an electrochemically detectable property; a sample application matrix; a blood cell separation matrix; a reagent or detection material storage matrix; a detection zone matrix; a suction matrix. Examples of these analytical elements are e.g. disclosed in US 2005/0118662 A1.

As outlined above, step ii. of the method comprises applying transfer foils on the front side and on the reverse side of the core strip intermediate product. The term "applying" as used herein is a broad term and is to be given its ordinary and customary meaning to a person of ordinary skill in the art and is not to be limited to a special or customized meaning. The term specifically may refer, without limitation, to a process of attaching, connecting, disposing and/or putting at least one element on at least one further element. The applying may comprise bringing at least two elements in contact with each other. Specifically, the applying in step ii. may comprise bringing at least one transfer foil in contact with each of the front side and the reverse side of the core strip intermediate product.

The term "transfer foil" as used herein is a broad term and is to be given its ordinary and customary meaning to a person of ordinary skill in the art and is not to be limited to a special or customized meaning. The term specifically may refer, without limitation, to at least one foil configured for carrying one or more elements engaged therewith. Specifically, the transfer foil may be configured for engaging with one or more elements and for carrying the engaged elements to another place or location. The transfer foil may be configured for reversibly engaging with the one or more elements. The transfer foil may be configured for releasing the one or more engaged elements, specifically at the other place or location to where they were conveyed by the transfer foil. The transfer foil may be a supporting product in the manufacturing method, specifically not being part of the manufactured plurality of analytical test elements.

The term "front side" as used herein is a broad term and is to be given its ordinary and customary meaning to a person of ordinary skill in the art and is not to be limited to a special or customized meaning. The term specifically may refer, without limitation, to an anterior surface of an object or element. The front side may specifically refer to a surface of an object or element being defined by particular features or elements disposed thereon or therein. Specifically, the front side of the core strip intermediate product may be a surface, in particular a surface of the carrier foil, having the analytical elements disposed thereon or therein. In other words, the front side of the core strip intermediate product may be a surface, in particular a surface of the carrier foil, facing towards the analytical elements attached to the carrier foil.

Consequently, the term "reverse side" as used herein is a broad term and is to be given its ordinary and customary meaning to a person of ordinary skill in the art and is not to be limited to a special or customized meaning and specifically may refer, without limitation, to a surface of an object or element opposite to the front side. The reverse side may specifically refer to a surface of an object or element being defined by an absence of the particular features or elements defining the front side of the object or element. The reverse side of the core strip intermediate product may be a surface, in particular a surface of the carrier foil, not having the analytical elements disposed thereon or therein. The reverse side of the core strip intermediate product may be a surface, in particular a surface of the carrier foil, facing away from the analytical elements attached to the carrier foil.

As outlined above, the transfer foil applied to the front side and the transfer foil applied to the reverse side engage with the core strips in an alternating fashion, the transfer foil on the front side and the core strips engaging with the transfer foil on the front side forming a first transfer intermediate product, the transfer foil on the reverse side and the core strips engaging with the transfer foil on the reverse side forming a second transfer intermediate product. The term "engage" as used herein is a broad term and is to be given its ordinary and customary meaning to a person of ordinary skill in the art and is not to be limited to a special or customized meaning. The term specifically may refer, without limitation, to a process of at least temporary connecting at least two elements with each other. Specifically, the engaging of the transfer foils with the core strips on the front side and the reverse side, respectively, in step ii. may comprise at least temporary connecting the transfer foils with the core strips. Thus, at a later point in time during the manufacturing method, the engagement of the transfer foils with the core strips may be released. The engaging in an alternating fashion may ensure that each of the core strips may only be connected to either the transfer foil on the front side or to the transfer foil on the reverse side.

The term "alternating fashion" as used herein is a broad term and is to be given its ordinary and customary meaning to a person of ordinary skill in the art and is not to be limited to a special or customized meaning. The term specifically may refer, without limitation, to a repeating mode of operation. Specifically, the engaging of the transfer foils on the front side and on the reverse side with the core strips in the alternating fashion may comprise a regular engaging of the of the transfer foils on the front side and on the reverse side with the core strips. For example, both the transfer foils applied to the front side and to the revere side may engage with every second core strip of the core strip intermediate product.

The term "transfer intermediate product" as used herein is a broad term and is to be given its ordinary and customary meaning to a person of ordinary skill in the art and is not to be limited to a special or customized meaning. The term specifically may refer, without limitation, to an intermediate product comprising transfer foils and core strips. Specifically, the transfer intermediate product may comprise at least one of the transfer foil on the front side and the transfer foil on the reverse side, and the core strips engaged therewith. The transfer intermediate product in its entirety may not be part of the manufactured plurality of analytical test elements but may be used in one or more following processing steps to manufacture the plurality of analytical test elements, specifically in at least one or more following separating and transferring method steps. Specifically, at least one part of the transfer intermediate product may not be part of the manufactured plurality of analytical test elements, such as the transfer foils, wherein at least one other part of the transfer intermediate product, such as the core strips, may be part the manufactured plurality of analytical test elements.

In general, the terms "first" and "second" as used herein are used for the purpose of nomenclature, only, without intention of ranking or numbering and without giving any preferences.

Further, the term "first transfer intermediate product" may refer, without limitation, to a transfer intermediate product having the transfer foil applied on top of the analytical elements of the core strips. In other words, in the first transfer intermediate product, the analytical elements of the core strip may be located in between the transfer foil and the carrier foil. In contrast, the term "second transfer intermediate product" may refer, without limitation, to a transfer intermediate product having the transfer foil applied on the side opposing the analytical elements of the core strips. Thus, in the second transfer intermediate product, the analytical elements may not be covered by the transfer foil. The second transfer intermediate product may comprise the carrier foil located in between the analytical elements and the transfer foil. The core strips comprised by the first and second transfer intermediate product may be spaced apart from each other by one width of the core strips.

As further outlined above, the method comprises in step iii. separating the first and second transfer intermediate product. The term "separating" as used herein is a broad term and is to be given its ordinary and customary meaning to a person of ordinary skill in the art and is not to be limited to a special or customized meaning. The term specifically may refer, without limitation, to a process of dividing or isolating two or more connected elements. Specifically, the separating in step iii. may comprise dividing or isolating the first and second transfer intermediate products from each other. The first and second transfer intermediate products may be handled independently from each other after the separating in step. iii.

Further, in step iv., as outlined above, the method comprises transferring the core strips onto at least one support foil, wherein the core strips, on the support foil, are spaced apart from each other by at least one width of one core strip. The term "transferring" as used herein is a broad term and is to be given its ordinary and customary meaning to a person of ordinary skill in the art and is not to be limited to a special or customized meaning. The term specifically may refer, without limitation, to a process of displacing, conveying or carrying one or more elements from one place or object to another. Specifically, the transferring in step iv. may comprise displacing the core strips from at least one transfer intermediate products onto the support foil. The transferring may comprise directly or indirectly displacing the core strips from the first and second transfer intermediate products onto the support foil. For example, step. iv. may comprise transferring the core strips directly from the first and second transfer intermediate products onto the support foil, specifically without further processing steps. For example, step iv. may comprise transferring the core strips indirectly from the first and second transfer intermediate products onto the support foil, wherein one or more further transfer steps, inversion steps or other processing steps may be comprised before and/or during step iv. before the core strips are finally transferred onto the support foil. Thus, in this case, the core strips in step iv. may be transferred from one or more of a third transfer intermediate product, a fourth transfer intermediate product and/or a fifth transfer intermediate product onto the support foil.

The term "width" as used herein is a broad term and is to be given its ordinary and customary meaning to a person of ordinary skill in the art and is not to be limited to a special or customized meaning. The term specifically may refer, without limitation, to an extension of an element in a direction essentially perpendicular to a longitudinal direction of extension. Specifically, the width of an element may be the elements' extension perpendicular to the longitudinal direction of extension of the element. For example, the element may be the core strip, specifically having an elongated or strip-shaped form, as outlined above. The width of the core strip may be the extension of the core strip in a direction perpendicular, i.e. taken at right angle, to a direction of extension of the elongated or strip-shaped form.

The method may specifically be performed as a continuous manufacturing process. The term "continuous" as used herein is a broad term and is to be given its ordinary and customary meaning to a person of ordinary skill in the art and is not to be limited to a special or customized meaning. The term specifically may refer, without limitation, to a characteristic of a process or object of being uninterrupted and/or perpetual.

Specifically, at least one of the carrier foil, the analytical test element, the core strip intermediate product, the transfer foil and the support foil may be provided as at least one web, specifically as at least as one web provided via at least one reel, more specifically as a continuous sheet, more specifically as a continuous belt or web, respectively. The term "web" as used herein is a broad term and is to be given its ordinary and customary meaning to a person of ordinary skill in the art and is not to be limited to a special or customized meaning. The term specifically may refer, without limitation, to a continuous sheet or roll of material.

Specifically, the method may be performed as a reel-to-reel process. The term "reel-to-reel process" as used herein is a broad term and is to be given its ordinary and customary meaning to a person of ordinary skill in the art and is not to be limited to a special or customized meaning. The term specifically may refer, without limitation, to a process involving at least two turning and/or rotating objects, such as at least two rotating wheels and/or rolls. The reel-to-reel process may specifically comprise transferring at least one element stored on a first roll, such as an element having a sheet form and/or tape form, onto a second roll, wherein at least one processing step is performed during the transfer. Thus, as an example, the reel-to-reel process may refer to an arbitrary process starting with a roll of material, e.g. with the first roll, and re-reeling after performing the process, such as into an output roll, e.g. into the second roll. The reel-to-reel-process may also be referred to as roll-to-roll-process. The method may further comprise at least one cutting process, wherein, in the cutting process, at least one part of at least one element is cut, specifically in a longitudinal direction. The at least one element is selected from the group consisting of: the core strip intermediate product; the first transfer intermediate product; the second transfer intermediate product. The term "cutting" as used herein is a broad term and is to be given its ordinary and customary meaning to a person of ordinary skill in the art and is not to be limited to a special or customized meaning. The term specifically may refer, without limitation, to a process of separating at least one object and/or element, wherein a defined and/or controlled edge and/or cutting edge is generated. The cutting may comprise using at least one cutting blade for cutting the at least one element in the cutting process, specifically in a cutting angle $\alpha$ between a surface of the element to be cut and a surface of the cutting blade, in particular at a cutting angle of $20° \leq \alpha \leq 40°$. The cutting process may specifically be a continuous cutting process. Additionally or alternatively, the method may comprise recycling at least a part of the material cut off during the cutting process, specifically material of the transfer foils.

The method may further comprise:

v. applying at least one further transfer foil on at least one of the first and second transfer intermediate products, such that the core strips are embedded in between the transfer foils, wherein the further transfer foil engages with every second of the core strips located on the at least one of the first and second transfer intermediate products, thereby generating a third transfer intermediate product; and

vi. removing the further transfer foil with the core strips engaging therewith from the third transfer intermediate product, such that the further transfer foil with the core strips engaging therewith form a fourth transfer intermediate product and that the remaining transfer foil of the third transfer intermediate product and the core strips engaging therewith after the removal of the further transfer foil form a fifth transfer intermediate product.

Steps. v. and vi. may specifically be performed prior to step iv., for example in between step iii. and step iv..

Again, in principle, the terms "third", "fourth" and "fifth" as used herein are used for the purpose of nomenclature, only, without intention of ranking or numbering and without giving any preferences.

Further, the term "third transfer intermediate product" may refer, without limitation, to a transfer intermediate product having the further transfer foil applied either on a side of the carrier foil facing away from the analytical elements of the core strips or on top of the analytical elements of the core strips. Specifically, in case the further transfer foil is applied to the first intermediate transfer product, the third transfer intermediate product may comprise the further transfer foil applied to the side of the carrier foil facing away from the analytical elements of the core strips of the first transfer intermediate product. In case the further transfer foil is applied to the second intermediate transfer product, the third transfer intermediate product may comprise the further transfer foil on top of the analytical elements of the core strips on the second transfer intermediate product. The third transfer intermediate product may have the core strips of the first or second transfer intermediate product located in between the transfer foil applied in step ii. and the further transfer foil applied in step v..

The term "fourth transfer intermediate product" may refer, without limitation, to a transfer intermediate product having the analytical elements of the core strips facing either away or towards the further transfer foil. Specifically, in case the fourth transfer intermediate product is obtained from the first intermediate transfer product having the further transfer foil applied thereto, the fourth transfer intermediate product may comprise the analytical elements of the core strips facing away from the further transfer foil. In case the fourth transfer intermediate product is obtained from the second intermediate transfer product having the further transfer foil applied thereto, the fourth transfer intermediate product may comprise the analytical elements of the core strips facing towards the further transfer foil.

Similarly, the term "fifth transfer intermediate product" may refer, without limitation, to a transfer intermediate product having the analytical elements of the core strips facing either away or towards the remaining transfer foil. Specifically, in case the fifth transfer intermediate product is obtained from the first intermediate transfer product having the further transfer foil applied thereto, the fifth transfer intermediate product may comprise the analytical elements of the core strips facing towards the remaining transfer foil. In case the fifth transfer intermediate product is obtained from the second intermediate transfer product having the further transfer foil applied thereto, the fifth transfer intermediate product may comprise the analytical elements of the core strips facing away from the remaining transfer foil.

In any case, the core strips comprised by the fourth and fifth transfer intermediate product may be spaced apart from each other by three widths of the core strips.

Steps v. and vi. may be performed repeatedly, wherein, after the first performing of steps v. and vi., step v. is performed on at least one of the fourth and fifth transfer intermediate products. By repeating performing steps v. and vi., the spacing of the core strips on the fourth and fifth transfer intermediate products can be further enlarged, in particular by a factor of 2 with every repetition of performing steps v. and vi..

The transfer foils may specifically comprise a plurality of through holes arranged in lines, specifically in lines oriented parallel to a longitudinal direction of extension of the respective transfer foil. The term "through hole" as used herein is a broad term and is to be given its ordinary and customary meaning to a person of ordinary skill in the art and is not to be limited to a special or customized meaning. The term specifically may refer, without limitation, to an arbitrary hollow structure extending completely through the material of an object. Specifically, the through hole may extend completely through the transfer foil, such as to provide an opening through the transfer foil. The through hole may be arbitrarily shaped. For example, the through hole may be a round hole. However, other forms of the through, such as a rectangular shapes, an elliptic shape, a polygonal shape or the like, are also feasible.

The method may further comprise applying at least one adhesive tape to the transfer foils in such a way that at least one adhesive region of the adhesive tape engages with the carrier foil of the core strips through the through holes. The term "adhesive tape" as used herein is a broad term and is to be given its ordinary and customary meaning to a person of ordinary skill in the art and is not to be limited to a special or customized meaning. The term specifically may refer, without limitation, to a strip or a band configured for binding to at least one separate element or object. Specifically, the adhesive tape may be configured for binding with and/or adhering to the carrier foil thereby engaging with the carrier foil.

Further, the core strips may be placed adjacent to each other on the core strip intermediate product having a pitch p, specifically a pitch corresponding to a width of the core strips. The term "pitch" as used herein is a broad term and is to be given its ordinary and customary meaning to a person of ordinary skill in the art and is not to be limited to a special or customized meaning. The term specifically may refer, without limitation, to a spatial repetition frequency or the distance between corresponding features in a periodic structure. The pitch may, as an example, be determined from center to center of repeating neighboring structures. For example, the pitch of the core strips on the core strip intermediate product may comprise a spacing of the core strips, specifically being determined from center to center of the core strips.

Further, the through holes of each line have a pitch s, with:

$$s = 2^m * p,$$

with m being a positive integer.

Each of the transfer foils may specifically comprise a plurality of at least two different lines of through holes, the at least two different lines of through holes differing from each other with respect to the pitch s of their through holes. The transfer foils may comprise at least one first line of through holes, specifically two first lines of through holes, with m = 1, and at least one second line of through holes, specifically two second lines of through holes, with m = 2, and, optionally, at least one third line of through holes, specifically two third lines of through holes, with m = 3.

Additionally or alternatively, in step ii., the transfer foils on the front side and on the reverse side may be offset in a longitudinal direction of the transfer foils by an offset distance of s/2, such that the transfer foils on opposing sides of the core strips, via their respective adhesive tapes, engage with the core strips in an alternating fashion.

The method may further comprise at least one edge cutting process. In the edge cutting process, at least one part of at least one of the transfer foils may be cut off, specifically in a longitudinal direction. At least one part being cut off may contain at least one line of through holes. The edge cutting process may be a continuous cutting process.

The core strip intermediate product may further comprise rim portions, specifically uncut rim portions, specifically rim portions extending in a longitudinal direction of extension along edges of the core strip intermediate product. The term "rim portion" as used herein is a broad term and is to be given its ordinary and customary meaning to a person of ordinary skill in the art and is not to be limited to a special or customized meaning. The term specifically may refer, without limitation, to at least one edge area of an object. The rim portion may specifically be at least one edge area of the core strip intermediate product, e.g. two edge areas limiting a size of the core strip intermediate in a direction perpendicular to the longitudinal direction of extension. The plurality of core strips may be located in between the rim portions. Further, before separating the first and second transfer intermediate products in step iii., the plurality of core strips may be held in place by the rim portions. The method, additionally or alternatively, may further comprise removing the rim portions, specifically cutting off the rim portions, before separating the first and second transfer intermediate product in step iii..

The method may further comprise:

vii. covering the core strips on the support foil with at least one cover sealing foil.

The term "cover" as used herein is a broad term and is to be given its ordinary and customary meaning to a person of ordinary skill in the art and is not to be limited to a special or customized meaning. The term specifically may refer, without limitation, to a process of applying at least one element onto at least one further element. Specifically, the covering of the core strips may comprise applying at least one element onto the core strips, the applied element being configured for sealing and/or protecting the covered core strips. The element applied onto the core strips may specifically be a foil, such as a metal foil, e.g. an aluminum foil. Consequently, the element applied to the core strips may be referred to as "cover sealing foil".

The method may further comprise applying perforation lines into the support foil with the core strips disposed thereon, and optionally also into the cover sealing foil, such that at least one perforation line is disposed in between neighboring core strips. The term "perforation line" as used herein is a broad term and is to be given its ordinary and customary meaning to a person of ordinary skill in the art and is not to be limited to a special or customized meaning. The term specifically may refer, without limitation, to a non-continuous course of element, e.g. holes, allowing separation of two or more sections neighboring the course of elements. The perforation line may specifically be a straight line of holes allowing separation of two analytical test elements neighboring the separation line. However, other forms, e.g. curved perforation lines, are also feasible.

The core strips may be placed adjacent to each other on the core strip intermediate product have a pitch p, specifically a pitch corresponding to a width of the core strips. On the support foil, the core strips may be spaced apart from each other by a separation distance d:

$$d = (2^n - 1)*p,$$

with n being a positive integer.

In other words, the core strips on the support foil may have a separation distance from each other, specifically, from neighboring core strips, wherein the separation distance may exceed the distance of the core strips on the core strip intermediate product.

The core strips each may have a contact side and, in step iv., may be applied to the support foil with their contact sides. The term "contact side" as used herein is a broad term and is to be given its ordinary and customary meaning to a person of ordinary skill in the art and is not to be limited to a special or customized meaning. The term specifically may refer, without limitation, to a side of the carrier foil facing away of the analytical elements of the corresponding core strip.

Specifically, in step iv., the core strips may be attached to the support foil by using a bonding method, specifically a bonding method selected from the group consisting of gluing, specifically by using hot melt adhesive; welding.

Alternatively or additionally, as described in further detail above, some of the transfer intermediate products may be embodied such that the contact sides of the core strips faced towards the transfer foils of the respective transfer intermediate products. In this case, the method may further comprise:

viii. inverting the core strips having contact sides facing towards their respective transfer foil, the inverting comprising applying an inversion transfer foil onto the core strips on a side opposing the contact sides of the core strips, the inverting further comprising removing the transfer foil from the contact sides of the core strips.

The term "inverting" as used herein is a broad term and is to be given its ordinary and customary meaning to a person of ordinary skill in the art and is not to be limited to a special or customized meaning. The term specifically may refer, without limitation, to a process of turning an element upside down. Specifically, after inverting an element, a top side of the element may be a down side and vice versa. The inverting of the core strips may result in having the contact sides facing towards their respective transfer foil. The inverting may comprise applying the inversion transfer foil, which specifically may be embodied identical to the transfer foil and/or the further transfer foil, onto the core strips of the respective transfer intermediate product, specifically on the analytical elements of the core strips, cutting at least one line of through holes of the inversion transfer foil and the transfer intermediate product and removing the transfer foil from the transfer intermediate product.

At least one of the transfer foils may comprise a plurality of positioning marks, specifically positioning marks arranged in a line, more specifically in a line perpendicular to a longitudinal direction of the transfer foil. The positioning marks may comprise through holes through the transfer foil. At least two lines of positioning marks may be located on opposing edges of the transfer foil. The positioning marks may be arranged in at least one line, specifically at least one line extending in a longitudinal direction of the transfer foil. The positioning marks, in the line, may have a pitch l, wherein the core strips being placed adjacent to each other on the core strip intermediate product may have a pitch p, with

$$p = q * l,$$

with q being a positive integer.

In a further aspect of the present invention, a manufacturing device for manufacturing a plurality of analytical, specifically diagnostic, test elements is disclosed, the analytical test elements comprising at least one core strip and at least a support foil strip.

The term "manufacturing device" as used herein is a broad term and is to be given its ordinary and customary meaning to a person of ordinary skill in the art and is not to be limited to a special or customized meaning. The term specifically may refer, without limitation, to an arbitrary device or set of interacting or interdependent devices configured for manufacturing, as defined above. The manufacturing device may be or may comprise single device or a set of at least two interacting or interdependent components or devices forming a whole, in order to fulfill at least one common function of manufacturing. The at least two devices may be handled independently or may be coupled or connectable. The manufacturing device may specifically be configured for performing the method of manufacturing a plurality of analytical test elements according to the present invention, such as according to any one of the embodiments disclosed above and/or according to any embodiment disclosed in further detail below. Thus, for definitions of terms and possible embodiments of the method or products, reference is made to the description of the method above.

The manufacturing device comprises:

I. at least one core strip intermediate product supply device configured for supplying at least one core strip intermediate product, specifically continuously, the core strip intermediate product comprising at least one carrier foil and at least one analytical element attached to the at least one carrier foil, the carrier foil and the attached analytical element, in the core strip intermediate product, being cut such that a plurality of core strips of the analytical test elements is defined, the core strips being placed adjacent to each other;

II. at least one transfer foil application device configured for applying transfer foils on a front side and on a reverse side of the core strip intermediate product, wherein the transfer foil applied to the front side and the transfer foil applied to the reverse side engage with the core strips in an alternating fashion, the transfer foil on the front side and the core strips engaging with the transfer foil on the front side forming a first transfer intermediate product, the transfer foil on the reverse side and the core strips engaging with the transfer foil on the reverse side forming a second transfer intermediate product;

III. at least one separation device configured for separating the first and second transfer intermediate products; and

IV. at least one transfer device configured for transferring the core strips onto at least one support foil, specifically continuously, such that the core strips, on the support foil, are spaced apart from each other by at least one width of one core strip.

The manufacturing device specifically may be configured for performing and/or implementing the method of manufacturing the plurality of analytical test elements according to the present invention, such as according to any one of the embodiments described above and/or according to any one of the embodiments described in further detail below. Thus, for possible options and/or definitions of the device and its components, reference may be made to the description of the method as given herein.

The term "core strip intermediate product supply device" as used herein is a broad term and is to be given its ordinary and customary meaning to a person of ordinary skill in the art and is not to be limited to a special or customized meaning. The term specifically may refer, without limitation, to an arbitrary device or set of interacting or interdependent devices configured for providing, as defined above. The core strip intermediate product supply device may specifically be configured for providing the core strip intermediate product.

The term "transfer foil application device" as used herein is a broad term and is to be given its ordinary and customary meaning to a person of ordinary skill in the art and is not to be limited to a special or customized meaning. The term specifically may refer, without limitation, to an arbitrary device or set of interacting or interdependent devices configured for applying, as defined above. The transfer foil application device may specifically be configured for applying the at least one of the transfer foils, the further transfer foil and the inversion transfer foil onto the respective intermediate product, in particular onto at least one of the core strip intermediate product or a transfer intermediate product.

The term "separation device" as used herein is a broad term and is to be given its ordinary and customary meaning to a person of ordinary skill in the art and is not to be limited to a special or customized meaning. The term specifically may refer, without limitation, to an arbitrary device or set of interacting or interdependent devices configured for separating, as defined above. The separation device may specifically be configured for separating at least one of the first and second transfer intermediate products, the further transfer foil from the third transfer intermediate product and the transfer foil from the core strips, specifically after transferring onto the support foil.

The term "transfer device" as used herein is a broad term and is to be given its ordinary and customary meaning to a person of ordinary skill in the art and is not to be limited to a special or customized meaning. The term specifically may refer, without limitation, to an arbitrary device or set of interacting or interdependent devices configured for transferring, as defined above. The transfer device may specifically be configured for transferring the core strips from at least one transfer intermediate product, in particular from at least one of the first and second transfer intermediate products, or the fourth and fifth transfer intermediate products, onto the support foil.

In a further aspect of the present invention, a plurality of analytical test elements is disclosed, the plurality of analytical test elements being produced by the method of manufacturing a plurality of analytical test elements according to the present invention, such as according to any one of the embodiments disclosed above and/or according to any one of the embodiments disclosed in further detail below. The plurality of analytical test elements produced by the manufacturing method according to the present invention may be identified by being assembled in units of manufactured plurality of analytical test elements, e.g. being assembled in roll ware units or sheet ware units of 10, 20, 50, or even 100 analytical test elements having the perforation lines in between neighboring analytical test elements. Additionally or alternatively, the plurality of analytical test elements produced by the manufacturing method according to the present invention may be identified at the cutting edge between two separations of plurality of analytical test elements, e.g. at a plain cutting edge for a batch manufacturing method or at an angled cutting edged for a continuous manufacturing method.

The manufacturing method and the manufacturing device according to the present invention may provide a larger number of advantages, specifically compared with known methods or devices. The manufacturing method and the manufacturing device according to the present invention, specifically in the continuous manufacturing process, e.g. performed as a reel-to-reel process, allow manufacturing disposables, i.e. the plurality of analytical test elements, continuously. Further, it may be possible to manufacture a plurality of analytical test elements as roll ware, wherein the core strips comprised by the manufactured plurality of analytical test elements may be spaced apart from each other by a defined distance. The cover sealing foil, e.g. an aluminum foil, may seal the core strips, specifically every core strips separately. The cover sealing foil and/or the support foil may comprise perforation lines such that the analytical test element may be isolated from the plurality of analytical test element by the user.

The manufacturing method and the manufacturing device according to the present invention may specifically require low device effort but nevertheless may be suitable for manufacturing the plurality of analytical test element at high quantities with high throughput and at low costs of production.

Summarizing and without excluding further possible embodiments, the following embodiments may be envisaged:

Embodiment 1. A method of manufacturing a plurality of analytical, specifically diagnostic, test elements comprising at least one core strip and at least one support foil strip, the method comprising:

i. providing at least one core strip intermediate product, the core strip intermediate product comprising at least one carrier foil and at least one analytical element attached to the at least one carrier foil, the carrier foil and the attached analytical element, in the carrier intermediate product, being cut such that a plurality of core strips of the analytical test elements is defined, the core strips being placed adjacent to each other;

ii. applying transfer foils on a front side and on a reverse side of the core strip intermediate product, wherein the transfer foil applied to the front side and the transfer foil applied to the reverse side engage with the core strips in an alternating fashion, the transfer foil on the front side and the core strips engaging with the transfer foil on the front side forming a first transfer intermediate product, the transfer foil on the reverse side and the core strips engaging with the transfer foil on the reverse side forming a second transfer intermediate product;

iii. separating the first and second transfer intermediate products; and

iv. transferring the core strips onto at least one support foil, wherein the core strips, on the support foil, are spaced apart from each other by at least one width of one core strip.

Embodiment 2. The method according to the preceding embodiment, wherein the method is performed as a continuous manufacturing process.

**Embodiment 3.** The method according to the preceding embodiment, wherein at least one of the carrier foil, the analytical test element, the core strip intermediate product, the transfer foil and the support foil are provided as at least one web, specifically as at least as one web provided via at least one reel, more specifically as a continuous sheet, more specifically as a continuous belt or web, respectively.

**Embodiment 4.** The method according to any one of the preceding embodiments, wherein the method is performed as a reel-to-reel process.

**Embodiment 5.** The method according to any one of the preceding embodiments, the method further comprising at least one cutting process, wherein, in the cutting process, at least one part of at least one element is cut, specifically in a longitudinal direction, wherein the at least one element is selected from the group consisting of the core strip intermediate product; the first transfer intermediate product; the second transfer intermediate product.

**Embodiment 6.** The method according to the preceding embodiment, wherein the cutting process is a continuous cutting process.

**Embodiment 7.** The method according to any one of the two preceding embodiments, the method comprising recycling at least a part of the material cut off during the cutting process, specifically material of the transfer foils.

**Embodiment 8.** The method according to any one of the preceding embodiments, further comprising:

> v. applying at least one further transfer foil on at least one of the first and second transfer intermediate products, such that the core strips are embedded in between the transfer foils, wherein the further transfer foil engages with every second of the core strips located on the at least one of the first and second transfer intermediate products, thereby generating a third transfer intermediate product; and
> vi. removing the further transfer foil with the core strips engaging therewith from the third transfer intermediate product, such that the further transfer foil with the core strips engaging therewith form a fourth transfer intermediate product and that the remaining transfer foil of the third transfer intermediate product and the core strips engaging therewith after the removal of the further transfer foil form a fifth transfer intermediate product.

**Embodiment 9.** The method according to the preceding embodiment, wherein steps v. and vi. are performed repeatedly, wherein, after the first performing of steps v. and vi., step v. is performed on at least one of the fourth and fifth transfer intermediate products.

**Embodiment 10.** The method according to any one of the preceding embodiments, wherein the transfer foils comprise a plurality of through holes arranged in lines, specifically in lines oriented parallel to a longitudinal direction of extension of the respective transfer foil, wherein the method further comprises applying at least one adhesive tape to the transfer foils in such a way that at least one adhesive region of the adhesive tape engages with the carrier foil of the core strips through the through holes.

**Embodiment 11.** The method according to the preceding embodiment, wherein the core strips being placed adjacent to each other on the core strip intermediate product have a pitch p, specifically a pitch corresponding to a width of the core strips, wherein the through holes of each line have a pitch s, with:

$$s = 2^m * p,$$

with m being a positive integer.

**Embodiment 12.** The method according to the preceding embodiment, wherein the transfer foils each comprise a plurality of at least two different lines of through holes, the at least two different lines of through holes differing from each other with respect to the pitch s of their through holes.

**Embodiment 13.** The method according to the preceding embodiment, wherein the transfer foils comprise at least one first line of through holes, specifically two first lines of through holes, with m = 1, and at least one second line of through holes, specifically two second lines of through holes, with m = 2, and, optionally, at least one third line of through holes, specifically two third lines of through holes, with m = 3.

**Embodiment 14.** The method according to any one of the three preceding embodiments, wherein, in step ii., the transfer foils on the front side and on the reverse side are offset in a longitudinal direction of the transfer foils by an offset distance of s/2, such that the transfer foils on opposing sides of the core strips, via their respective adhesive tapes, engage with the core strips in an alternating fashion.

**Embodiment 15.** The method according to any one of the five preceding embodiments, the method further comprising at least one edge cutting process, wherein, in the edge cutting process, at least one part of at least one of the transfer foils is cut off, specifically in a longitudinal direction, wherein the at least one part being cut off contains at least one line of through holes.

Embodiment 16. The method according to the preceding embodiment, wherein the edge cutting process is a continuous cutting process.

Embodiment 17. The method according to anyone of the preceding embodiments, wherein step i. comprises attaching the at least one analytical element to the at least one carrier foil, and further cutting the carrier foil and the attached analytical element such that the plurality of core strips is formed.

Embodiment 18. The method according to any one of the preceding embodiments, wherein the core strip intermediate product has a longitudinal direction of extension, specifically a longitudinal direction of extension of a carrier foil web, wherein the core strip intermediate product is cut such that the plurality of core strips of the analytical test elements is oriented essentially perpendicular to the longitudinal direction of extension.

Embodiment 19. The method according to any one of the preceding embodiments, the core strip intermediate product being cut such that the plurality of core strips of the analytical test elements is defined by a plurality of parallel cutting lines.

Embodiment 20. The method according to any one of the preceding embodiments, wherein the core strip intermediate product further comprises rim portions, specifically uncut rim portions, specifically rim portions extending in a longitudinal direction of extension along edges of the core strip intermediate product, wherein the plurality of core strips is located in between the rim portions.

Embodiment 21. The method according to the preceding embodiment, wherein, before separating the first and second transfer intermediate products in step iii., the plurality of core strips is held in place by the rim portions.

Embodiment 22. The method according to any one of the two preceding embodiments, further comprising removing the rim portions, specifically cutting off the rim portions, before separating the first and second transfer intermediate product in step iii..

Embodiment 23. The method according to any one of the preceding embodiments, further comprising: vii. covering the core strips on the support foil with at least one cover sealing foil.

Embodiment 24. The method according to any one of the preceding embodiments, the method further comprising applying perforation lines into the support foil with the core strips disposed thereon, and optionally also into the cover sealing foil, such that at least one perforation line is disposed in between neighboring core strips.

Embodiment 25. The method according to any one of the preceding embodiments, wherein the core strips being placed adjacent to each other on the core strip intermediate product have a pitch p, specifically a pitch corresponding to a width of the core strips, wherein, on the support foil, the core strips are spaced apart from each other by a separation distance d:

$$d = (2^n - 1)*p,$$

with n being a positive integer.

Embodiment 26. The method according to any one of the preceding embodiments, wherein the at least one analytical element is selected from the group consisting of: a spreading fleece for receiving at least one liquid sample, specifically at least one liquid sample of a bodily fluid and/or at least one liquid sample obtained from a bodily fluid; at least one detection material, the detection material being configured for changing the at least one measurable property in the presence of at least one analyte to be detected, specifically at least one of an optically detectable property and an electrochemically detectable property; a sample application matrix; a blood cell separation matrix; a reagent or detection material storage matrix; a detection zone matrix; a suction matrix.

Embodiment 27. The method according to any one of the preceding embodiments, wherein the core strips each have a contact side and, in step iv., are applied to the support foil with their contact sides.

Embodiment 28. The method according to the preceding embodiment, wherein, in step iv., the core strips are attached to the support foil by using a bonding method, specifically a bonding method selected from the group consisting of: gluing, specifically by using hot melt adhesive; welding.

Embodiment 29. The method according to any one of the two preceding embodiments, wherein some of the transfer intermediate products are embodied such that the contact sides of the core strips faced towards the transfer foils of the respective transfer intermediate products, wherein the method further comprises:

viii. inverting the core strips having contact sides facing towards their respective transfer foil, the inverting comprising applying an inversion transfer foil onto the core strips on a side opposing the contact sides of the core strips, the inverting further comprising removing the transfer foil from the contact sides of the core strips.

Embodiment 30. The method according to any one of the preceding embodiments, wherein at least one of the transfer foils comprises a plurality of positioning marks, specifically positioning marks arranged in a line, more specifically in a line perpendicular to a longitudinal direction of the transfer foil.

Embodiment 31. The method according to the preceding embodiment, wherein the positioning marks comprise through holes through the transfer foil.

Embodiment 32. The method according to any one of the two preceding embodiments, wherein at least two lines of positioning marks are located on opposing edges of the transfer foil.

Embodiment 33. The method according to any one of the three preceding embodiments, wherein the positioning marks are arranged in at least one line, specifically at least one line extending in a longitudinal direction of the transfer foil, wherein the positioning marks, in the line, have a pitch l, wherein the core strips being placed adjacent to each other on the core strip intermediate product have a pitch p, with

$$p = q * l,$$

with q being a positive integer.

Embodiment 34. A manufacturing device for manufacturing a plurality of analytical, specifically diagnostic, test elements comprising at least one core strip and at least a support foil strip, wherein the manufacturing device comprises:

I. at least one core strip intermediate product supply device configured for supplying at least one core strip intermediate product, specifically continuously, the core strip intermediate product comprising at least one carrier foil and at least one analytical element attached to the at least one carrier foil, the carrier foil and the attached analytical element, in the core strip intermediate product, being cut such that a plurality of core strips of the analytical test elements is defined, the core strips being placed adjacent to each other;

II. at least one transfer foil application device configured for applying transfer foils on a front side and on a reverse side of the core strip intermediate product, wherein the transfer foil applied to the front side and the transfer foil applied to the reverse side engage with the core strips in an alternating fashion, the transfer foil on the front side and the core strips engaging with the transfer foil on the front side forming a first transfer intermediate product, the transfer foil on the reverse side and the core strips engaging with the transfer foil on the reverse side forming a second transfer intermediate product;

III. at least one separation device configured for separating the first and second transfer intermediate products; and

IV. at least one transfer device configured for transferring the core strips onto at least one support foil, specifically continuously, such that the core strips, on the support foil, are spaced apart from each other by at least one width of one core strip.

Embodiment 35. The manufacturing device according to the preceding embodiment, wherein the manufacturing device is configured for performing the method according to any one of the preceding embodiments referring to a method of manufacturing a plurality of analytical test elements.

Embodiment 36. A plurality of analytical test elements, the plurality of analytical test elements being produced by the method according to any one of the preceding embodiments referring to a method of manufacturing a plurality of analytical test elements.

Short description of the Figures

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.

In the Figures:

Figure 1    shows an embodiment of a manufacturing device according to the present invention in a schematic view;

Figure 2    shows a flow chart of an embodiment of a method of manufacturing a plurality of analytical test elements according to the present invention; and

Figures 3A to 3U    show exemplary intermediate products and exemplar analytical test elements.

Detailed description of the embodiments

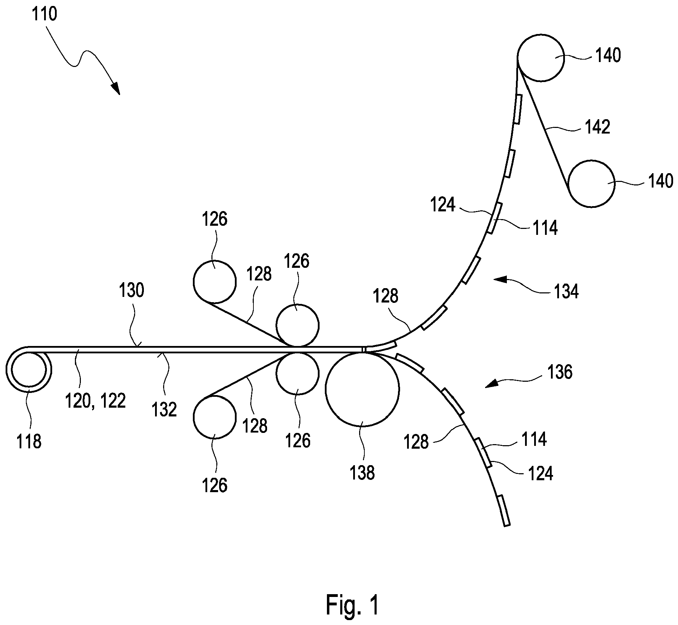

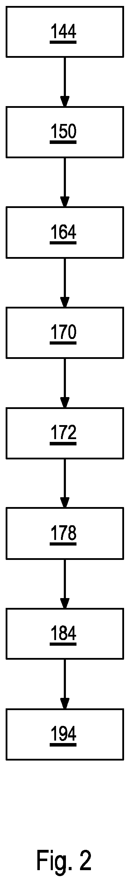

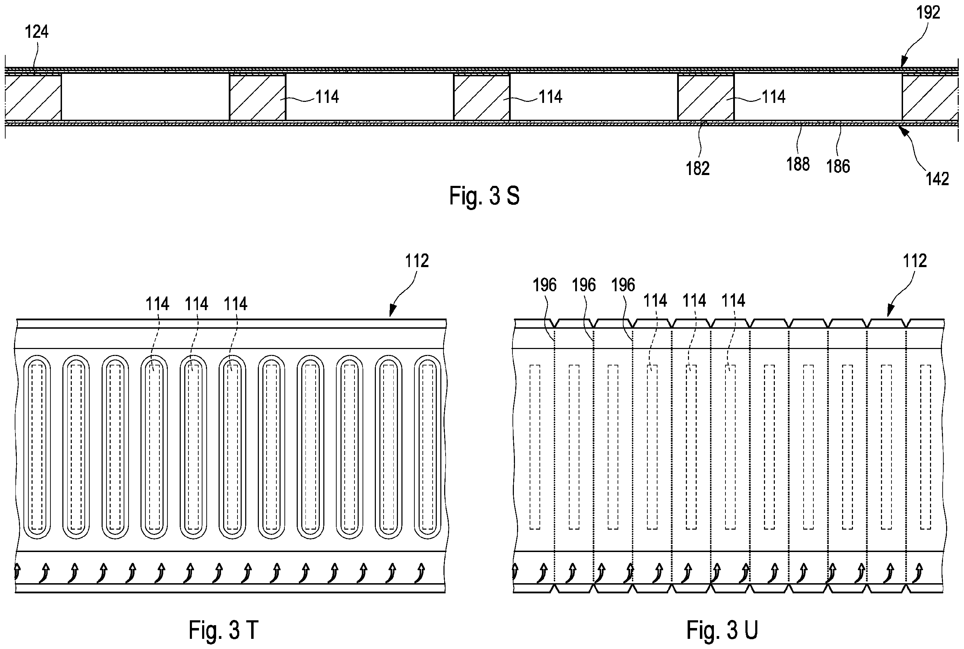

Figure 1 shows an exemplary embodiment of a manufacturing device 110 for manufacturing a plurality of analytical, specifically diagnostic, test elements 112 (see Figures 3T and 3U) in a schematic view, the analytical test elements 112 comprising at least one core strip 114 and at least a support foil strip 116. The manufacturing device 110 may specifically be configured for performing the method of manufacturing a plurality of analytical test elements 112 according to the present invention, such as according to the embodiment shown in Figure 2 and/or according to any other embodiment disclosed herein. Thus, for a detailed description of the manufacturing method, reference is made to the description of Figure 2.

The manufacturing device 110 comprises at least one core strip intermediate product supply device 118 configured for supplying at least one core strip intermediate product 120, specifically continuously, the core strip intermediate product 120 comprising at least one carrier foil 122 and at least one analytical element 124 attached to the at least one carrier foil 122, the carrier foil 122 and the attached analytical element 124, in the core strip intermediate product 120, being cut such that a plurality of core strips of the analytical test elements 112 is defined, the core strips 114 being placed adjacent to each other. In Figure 1, the cutting lines in the core strip intermediate product 120 supplied by the core strip intermediate product supply device 118 may not be visible since a width of the cutting lines may be minimal compared to the width of one core strip 116.

The manufacturing device 110 further comprises at least one transfer foil application device 126 configured for applying transfer foils 128 on a front side 130 and on a reverse side 132 of the core strip intermediate product 120, wherein the transfer foil 128 applied to the front side 130 and the transfer foil 128 applied to the reverse side 132 engage with the core strips 114 in an alternating fashion, the transfer foil 128 on the front side 130 and the core strips 114 engaging with the transfer foil 128 on the front side 130 forming a first transfer intermediate product 134, the transfer foil 128 on the reverse side 132 and the core strips 114 engaging with the transfer foil 128 on the reverse side 132 forming a second transfer intermediate product 136.

The manufacturing device 110 further comprises at least one separation device 138 configured for separating the first 134 and second transfer intermediate products 136. The manufacturing device 110 further comprises at least one transfer device 140 configured for transferring the core strips 114 onto at least one support foil 142, specifically continuously, such that the core strips 114, on the support foil 142, are spaced apart from each other by at least one width of one core strip 114. Before separating the first 134 and second transfer intermediate products 136, the manufacturing of the plurality of analytical test elements 112 may comprise at least one cutting step, as will be outlined in further detail below, specifically comprising cutting the core strip intermediate product 120. The cutting of the core strip intermediate product 120 may be performed previous to the first 134 and second transfer intermediate products 136 separation step performed by the separation device 138.

In the example of Figure 1, the manufacturing device 110 may be configured for performing the manufacturing method as a continuous manufacturing process, specifically as a reel-to-reel process. Thus, in this example, the devices of the manufacturing device 110 may be or may comprise reels for performing the respective manufacturing step

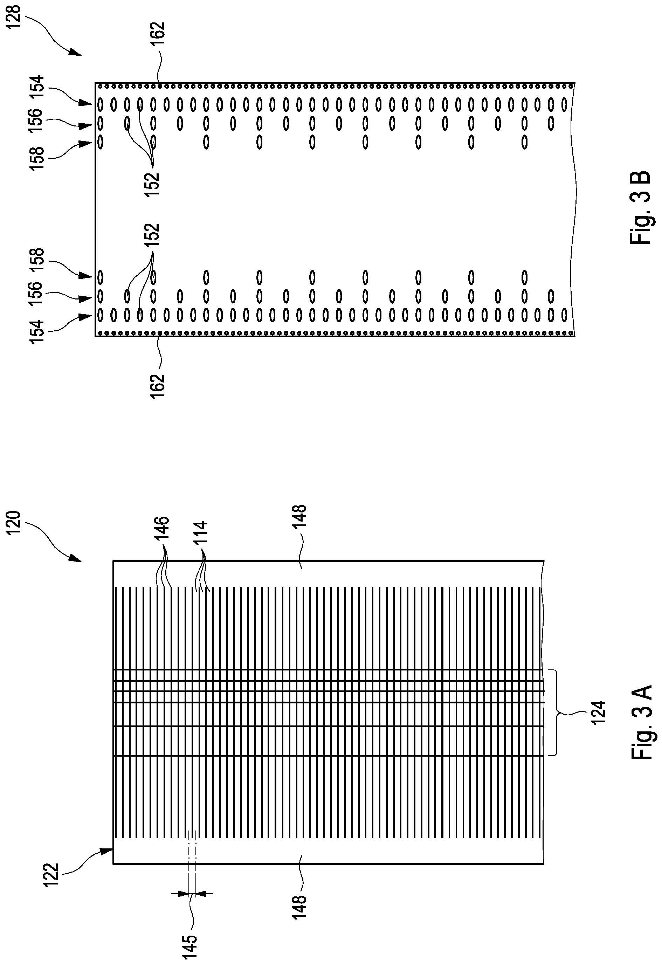

Figure 2 shows a flow chart of an exemplary embodiment of a method of manufacturing a plurality of analytical test elements 112 according to the present invention, the analytical test elements 112 comprising at least one core strip 114 and at least one support foil strip 116. Figures 3A to 3U show exemplary intermediate products and exemplary analytical test elements 112 obtained during performing the manufacturing method. Thus, in the following Figures 2 to 3U are described in conjunction.

The method comprises the steps shown in Figure 2, which, as an example, may be performed in the given order. It shall be noted, however, that a different order is also possible. Further, it is possible to perform one or more or even all of the method steps once or repeatedly. Further, it is possible to perform two or more of the method steps simultaneously or in a timely overlapping fashion. The method may comprise additional method steps, which are not listed. Further, it may be possible to only perform steps i. to iv. of the manufacturing method.

The method comprises providing at least one core strip intermediate product 120 (denoted by reference number 144). An exemplary embodiment of the core strip intermediate product 120 is shown in Figure 3A in a top view. As can be seen in Figure 3A, the core strip intermediate product 120 comprises the at least one carrier foil 122 and the at least one analytical element 124 attached to the at least one carrier foil 122. In the exemplary embodiment of the core strip intermediate product 120 shown in Figure 3A, multiple analytical elements 124 may be attached next to each other to the at least one carrier foil 122. By attaching various different analytical elements 124, an analytical assay can be performed by the series of analytical elements 124 when a liquid sample is applied, in particular by attaching analytical elements 124 selected from the group consisting of: a spreading fleece for receiving at least one liquid sample; at least one detection material, the detection material being configured for changing the at least one measurable property in the presence of at least one analyte to be detected; a sample application matrix; a blood cell separation matrix; a reagent or detection material storage matrix; a detection zone matrix; a suction matrix, in an interconnected manner at the at least one carrier foil 122. The carrier foil 122 and the attached at least one analytical element 124, in the carrier intermediate product 120, being cut such that a plurality of core strips 114 of the analytical test elements 112 is defined, the core strips 114 being placed adjacent to each other. The core strips 114 may specifically be placed adjacent to each other on the core strip intermediate product 120 have a pitch p (denoted by reference number 145), specifically a pitch corresponding to a width of the core strips 114.

Further, as shown in Figure 3A, the core strip intermediate product 120 may have a longitudinal direction of extension, specifically a longitudinal direction of extension of a carrier foil web. The core strip intermediate product 120 may be cut such that the plurality of core strips 114 of the analytical test elements 112 is oriented essentially perpendicular to the longitudinal direction of extension. The core strip intermediate product 120 may be cut such that the plurality of core strips 114 of the analytical test elements 112 is defined by a plurality of parallel cutting lines 146. Additionally, in this example, the core strip intermediate product 120 may further comprise rim portions 148, specifically uncut rim portions, specifically rim portions 148 extending in a longitudinal direction of extension along edges of the core strip intermediate product 120. The plurality of core strips 114 may be located in between the rim portions 148.

The method further comprises applying transfer foils 128 on the front side 130 and on the reverse side 132 of the core strip intermediate product 120 (denoted by reference number 150), wherein the transfer foil 128 applied to the front side 130 and the transfer foil 128 applied to the reverse side 132 engage with the core strips 114 in an alternating fashion, the transfer foil 128 on the front side 130 and the core strips 114 engaging with the transfer foil 128 on the front side 130 forming the first transfer intermediate product 134, the transfer foil 128 on the reverse side 132 and the core strips 114 engaging with the transfer foil 128 on the reverse side 132 forming the second transfer intermediate product 136.

An exemplary embodiment of the transfer foil 128 is shown in Figure 3B in a top view. As can be seen, the transfer foils 128 may comprise a plurality of through holes 152 arranged in lines, specifically in lines oriented parallel to a longitudinal direction of extension of the respective transfer foil. The through holes 152 of each line have a pitch s, with:

$$ s = 2^m * p, $$

with m being a positive integer.

Each of the transfer foils 128 may specifically comprise a plurality of at least two different lines of through holes 152, the at least two different lines of through holes 152 differing from each other with respect to the pitch s of their through holes 152. For example, in the exemplary embodiment of Figure 3B, the transfer foils 128 may comprise at least one first line 154 of through holes 152, specifically two first lines 154 of through holes 152, with m = 1, and at least one second line 156 of through holes 152, specifically two second lines 156 of through holes 152, with m = 2, and at least one third line 158 of through holes 152, specifically two third lines 158 of through holes 152, with m = 3.

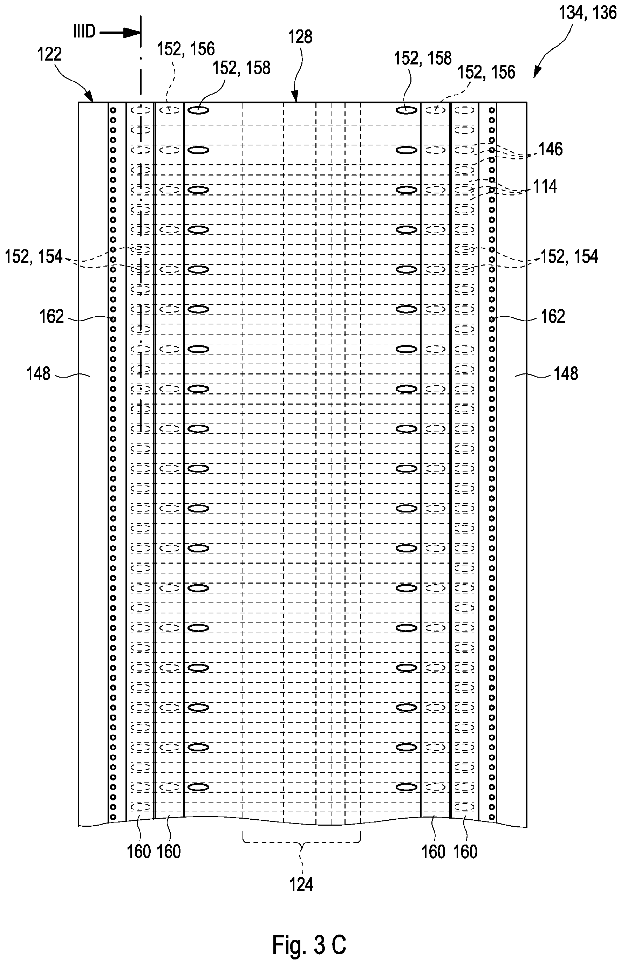

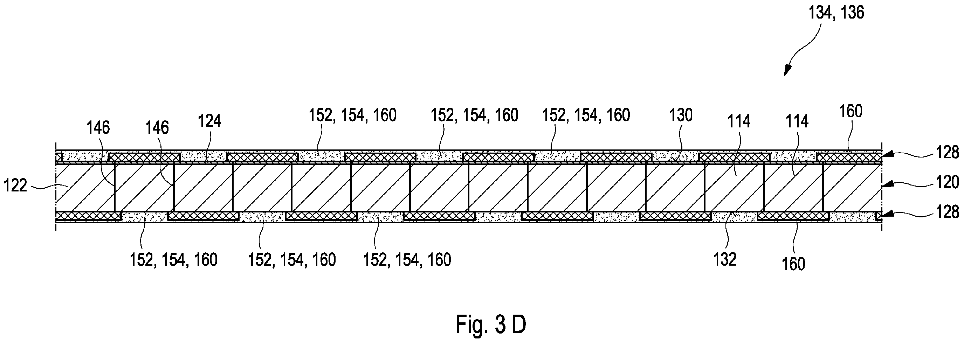

The method may further comprise, e.g. in step ii., applying at least one adhesive tape 160 to the transfer foils 128 in such a way that at least one adhesive region of the adhesive tape 160 engages with the carrier foil 122 of the core strips 114 through the through holes 152. An exemplary embodiment of the first transfer intermediate product 134 or the second transfer intermediate product 136 can be seen in Figure 3C in a top view. Specifically, Figure 3C shows another exemplary embodiment of the transfer foil 128 applied on the core strip intermediate product 120 to form the first transfer intermediate product 134 or the second transfer intermediate product 136. In the following, the exemplary embodiment of the transfer foil 128 shown in Figure 3C will be used. The corresponding first 134 or second transfer intermediate product 136 to the example of Figure 3C may comprise the respective transfer foil 128 offset in a longitudinal direction of the transfer foil 128 by an offset distance of s/2, such that the transfer foils 128 on opposing sides of the core strips 114, via their respective adhesive tapes 160, specifically accessible through the respective through holes 152, engage with the core strips 114 in an alternating fashion. This can be seen in Figure 3D. Figure 3D shows the first 134 or second transfer intermediate product 136 in a schematic side cut view along the first line 154 of through holes 152, as indicated in Figure 3C by the cutting line marked as III D. As shown in Figure 3D, the transfer foil 128 applied to the front side 130 of the core strip intermediate product 120 may engage with every second of the core strips 114 via the respective adhesive tape 160 through the through holes 152, wherein the transfer foil 128 applied to the reverse side 132 of the core strip intermediate product 120 may be offset by the offset distance of s/2, specifically in relation to the transfer foil 128 applied to the front side 130 of the core strip intermediate product 120, and, thus, may engage with the remaining core strips 114 via the respective adhesive tape 160 through the through holes 152.

Further, as can be seen in Figure 3C, at least one of the transfer foils 128, specifically both transfer foils 128 applied to the front side 130 and to the reverse side 132, may comprise a plurality of positioning marks 162, specifically positioning marks 162 arranged in a line, more specifically in a line perpendicular to a longitudinal direction of the transfer foil 128. The positioning marks 162 may comprise through holes through the transfer foil 128. At least two lines of positioning marks 162 may be located on opposing edges of the transfer foil 128. The positioning marks 162 may be arranged in at least one line, specifically at least one line extending in a longitudinal direction of the transfer foil 128. The positioning marks 162, in the line, may have a pitch l, wherein the core strips 114 being placed adjacent to each other on the core strip intermediate product 120 may have a pitch p, with

$$p = q * l,$$

with q being a positive integer.

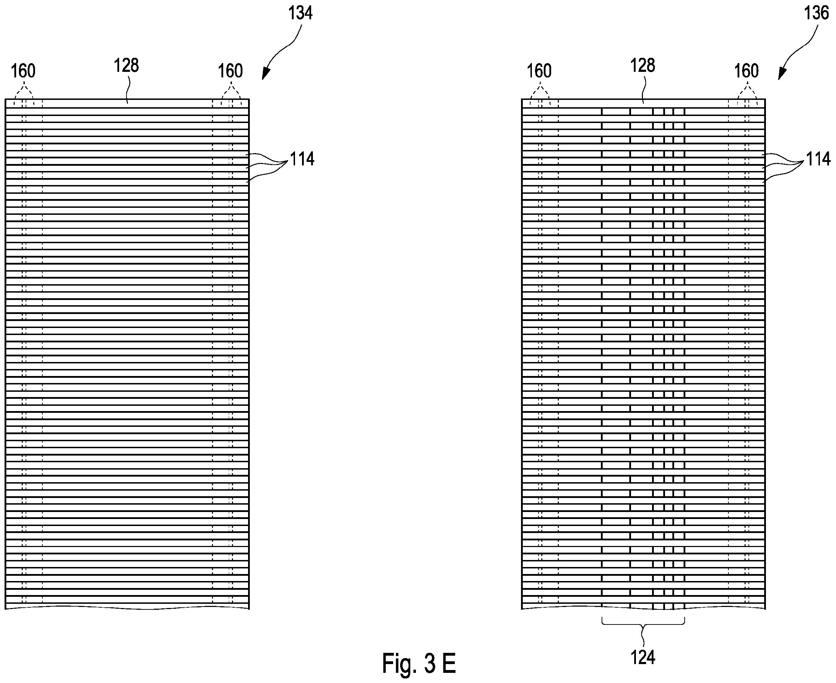

The method further comprises separating the first 134 and second transfer intermediate products 136 (denoted by reference number 164). Specifically, in this example, before separating the first 134 and second transfer intermediate products 136 in step iii., the plurality of core strips 114 may be held in place by the rim portions 148. The method may further comprise removing the rim portions 148, specifically cutting off the rim portions 148, before separating the first 134 and second transfer intermediate product 136 in step iii.. Figure 3E shows exemplary embodiments of the separated first 134 and second transfer intermediate products 136 in a top view. As for the first transfer intermediate product 134 (shown in the left part of Fig. 3E) the analytical elements 124 comprised by the core strips 114 are facing away from the viewer's perspective, wherein the analytical elements 124 comprised by the core strips 114 of the second transfer intermediate product 136 (shown in the right part of Fig. 3E) are facing towards the viewer's perspective. Thus, the analytical elements 124 of the first transfer intermediate product 134 are not visible in the view shown in Fig. 3E.

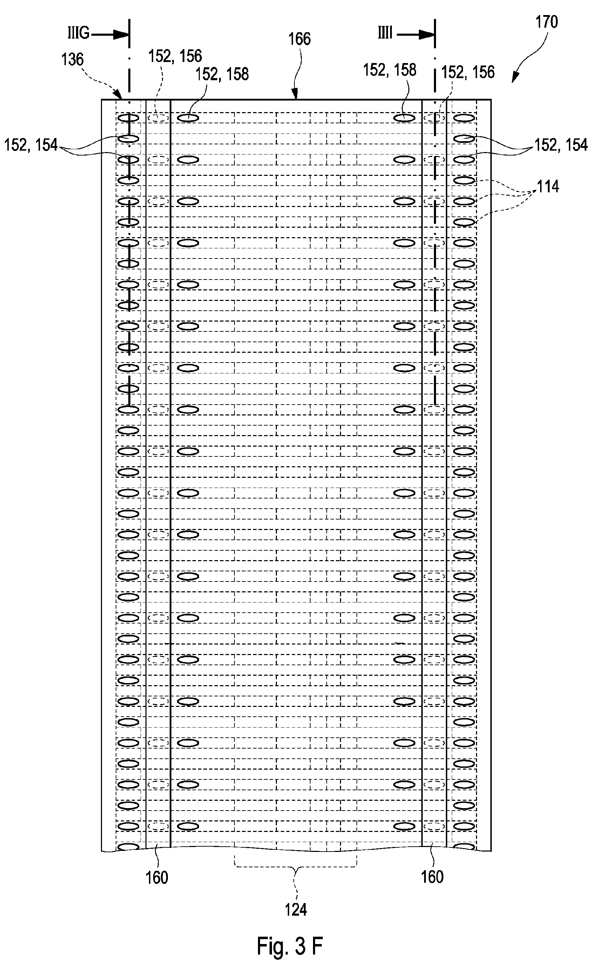

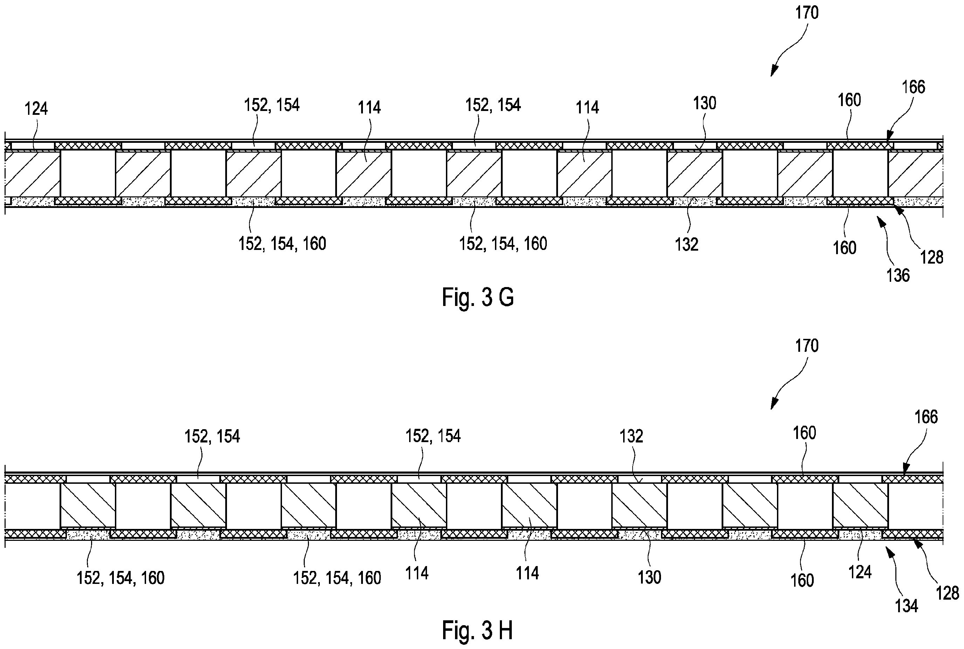

The method may further comprise applying at least one further transfer foil 166 on at least one of the first 134 and second transfer intermediate products 136 (denoted by reference number 168), such that the core strips 114 are embedded in between the transfer foils 128, 166, wherein the further transfer foil 166 engages with every second of the core strips 114 located on the at least one of the first 134 and second transfer intermediate products 136, thereby generating a third transfer intermediate product 170. Exemplary embodiments of the third transfer intermediate product 170 are shown in Figures 3F, 3G and 3H. Specifically, Figure 3F shows a top view of a third transfer intermediate product 170. Figure 3G shows the third transfer intermediate product 170 obtained by applying the further transfer foil 166 on the second transfer intermediate products 136 in a schematic side cut view along the first line 154 of through holes 152, as indicated by the cutting line marked as III G through the first line 154 in Figure 3F. Figure 3H shows the third transfer intermediate product 170 obtained by applying the further transfer foil 166 on the first transfer intermediate products 134 in a schematic side cut view along the first line 154 of through holes 152, as indicated by the cutting line marked as III G through the first line 154 in Figure 3F. As can be seen in Figures 3G and 3H, the third transfer intermediate products 170 obtained from the first 134 and second transfer intermediate products 136 may be reversed with respect to each other.

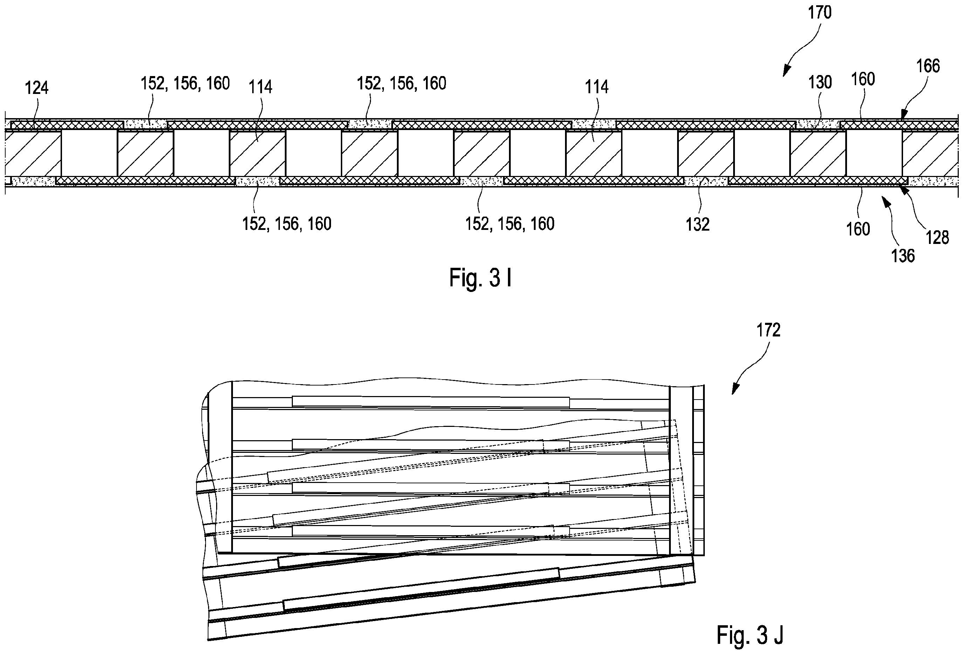

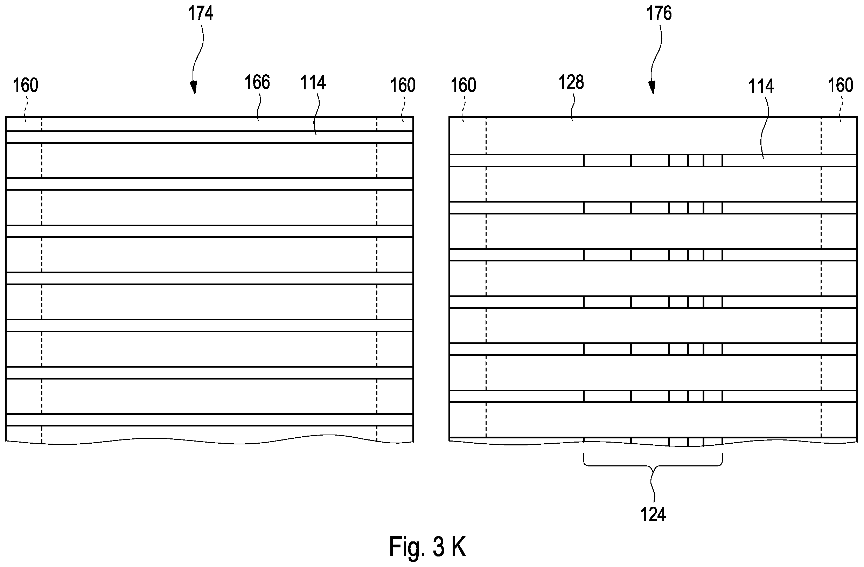

The method may further comprise removing the further transfer foil 166 with the core strips 114 engaging therewith from the third transfer intermediate product 170 (denoted by reference number 172), such that the further transfer foil 166 with the core strips 114 engaging therewith form a fourth transfer intermediate product 174 and that the remaining transfer foil 128 of the third transfer intermediate product 170 and the core strips 114 engaging therewith after the removal of the further transfer foil 166 form a fifth transfer intermediate product 176. Similar to step iii., prior to removing the further transfer foil 166, the method may comprise at least one edge cutting process. In the edge cutting process, at least one part of at least one of the transfer foils 128 may be cut off, specifically in a longitudinal direction. At least one part being cut off may contain at least one line of through holes 152. In this example, separating the fourth 174 and fifth transfer intermediate products 176 may comprise cutting off the first lines 154 of through holes 152. The removing of the further transfer foil 166 is exemplarily shown in Figures 3I and 3J. Figure 3I shows an exemplary embodiment of the third transfer intermediate product 170 after cutting off the first lines 154 of through holes 152 in a schematic side cut view along the second line 156 of through holes 152, as indicated by the cutting line marked as III I through the second line 156 in Figure 3F. After cutting, the further transfer foil 166 can be removed with the core strips 114 engaging therewith, as exemplarily shown in Figure 3J in a top-perspective view. Exemplary embodiments of the obtained fourth transfer intermediate product 174 and fifth transfer intermediate product 176 are shown in Figure 3K in a top view. As can be seen in Figure 3K, the fifth transfer intermediate product 176 may comprise the analytical elements 124 of the core strips 114 facing away from the remaining transfer foil 128. As for the forth transfer intermediate product 174 (shown in the left part of Fig. 3K) the analytical elements 124 comprised by the core strips 114 are facing away from the viewer's perspective, wherein the analytical elements 124 comprised by the core strips 114 of the fifth transfer intermediate product 176 (shown in the right part of Fig. 3K) are facing towards the viewer's perspective. Thus, the analytical elements 124 of the forth transfer intermediate product 174 are not visible in the view shown in Fig. 3K.

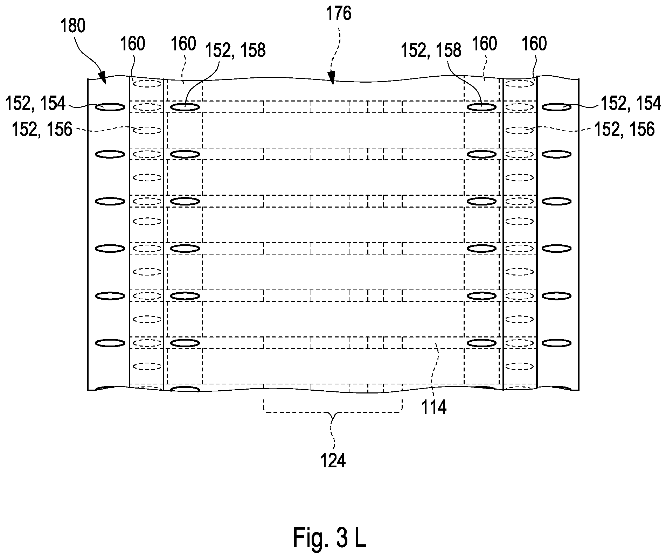

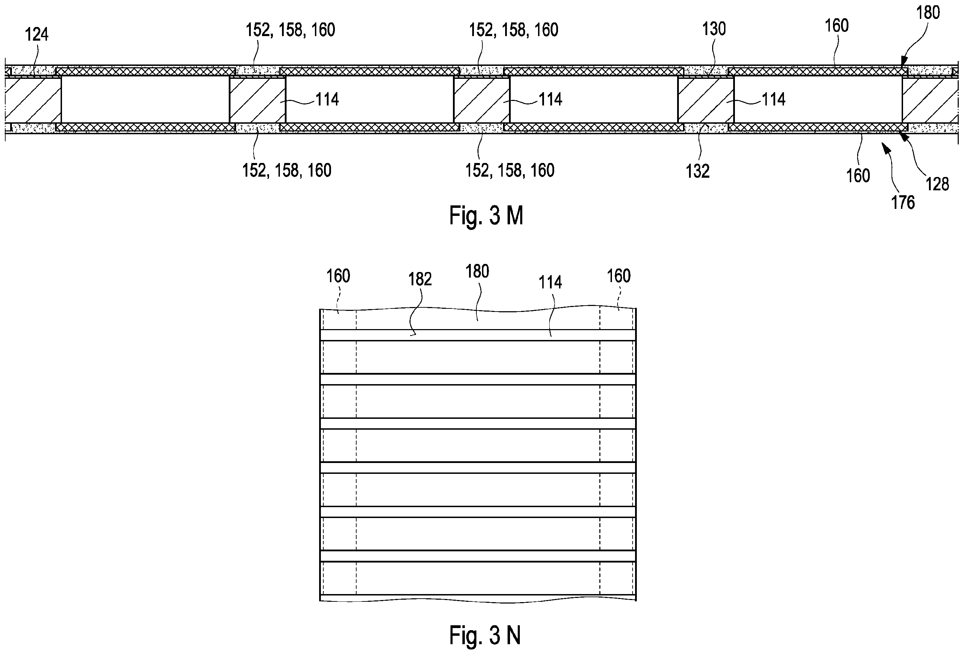

Thus, in this exemplary embodiment, the method may further comprise inverting the core strips 114 having contact sides facing towards their respective transfer foil 128, specifically inverting the core strips 114 of the fifth transfer intermediate product 176 (denoted by reference number 178). Alternatively, e.g. in case the core strips 114 may be directly transferred to the support foil 142 from the first 134 and second transfer intermediate product 136, the method may comprise inverting the core strips 114 having contact sides facing towards their respective transfer foil 128, specifically inverting the core strips 114 of the second transfer intermediate product 136 (not shown in the Figures). The inverting may comprise applying an inversion transfer foil 180 onto the core strips 114 on a side opposing the contact sides of the core strips 114, the inverting further comprising removing the transfer foil 128 from the contact sides of the core strips 114. The inverting may specifically comprise applying the inversion transfer foil 180, which specifically may be embodied identical to the transfer foil 128 and/or the further transfer foil 166, onto the core strips 114 of the fifth transfer intermediate product 176. An exemplary embodiment of the inversion transfer foil 180 applied on the fifth transfer intermediate product 176 is shown in Figures 3L and 3M. Figure 3L shows an exemplary embodiment the fifth transfer intermediate product 176 and the applied inversion transfer foil 180 in a top view, wherein Figure 3M shows an exemplary embodiment the fifth transfer intermediate product 176 and the applied inversion transfer foil 180 in a schematic side cut view along the third line 158 of through holes 152.

The inverting may further comprise cutting at least one line of the through holes 152 of the inversion transfer foil 180, specifically cutting the second line 156 of the through holes 152 of the inversion transfer foil 180 and removing the transfer foil 128 from the fifth transfer intermediate product 176. An exemplary result of the inverting step is shown in Figure 3N in a top view. Figure 3N shows the inversion transfer foil 180 engaging with the core strips 114, wherein the contact sides 182 may face away from the inversion transfer foil 180.

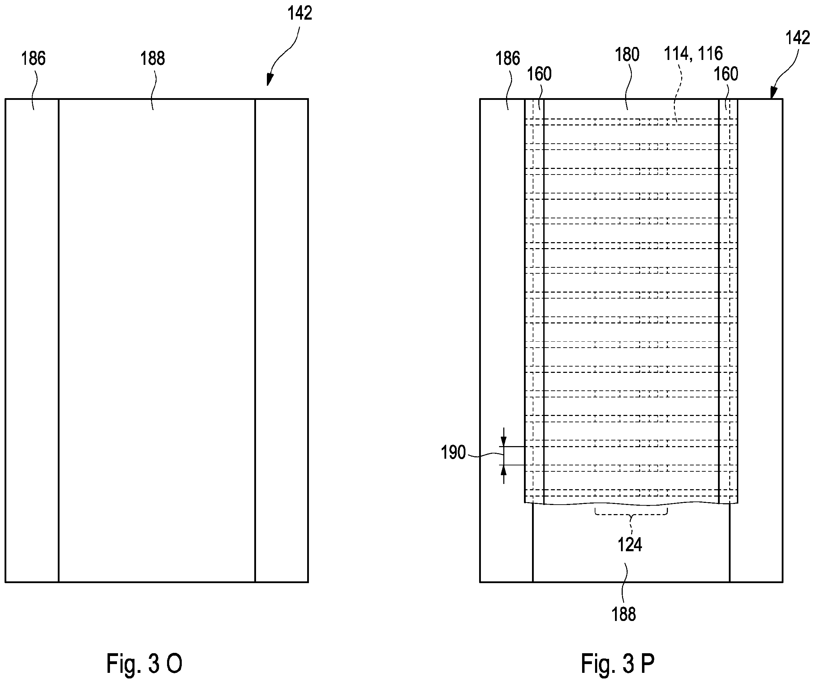

Turning back to Figure 2, the method further comprises transferring the core strips 114 onto at least one support foil 142 (denoted by reference number 184), wherein the core strips 114, on the support foil 142, are spaced apart from each other by at least one width of one core strip 114. An exemplary embodiment of the support foil 142 is shown in Figure 3O in a top view. In this example, the support foil 142 may comprise at least one aluminum foil 186 and at least one adhesive foil 188 applied thereto. An exemplary embodiment of the core strips 114 transferred to the support foil 142 is shown in Figure 3P in a top view. As can be seen in Figure 3P, the analytical elements 124 of the core strips 114 may face away from the support foil 142.

Further, as can be seen in Figures 3A and 3P, the core strips 114 may be placed adjacent to each other on the core strip intermediate product 120 having a pitch p, specifically a pitch corresponding to a width of the core strips 114 (see Figure 3A). On the support foil 142 (see Figure 3P), the core strips 114 may be spaced apart from each other by a separation distance d (denoted by reference number 190):

$$d = (2^n - 1)*p,$$

with n being a positive integer.

In this example, the separation distance may be d = 3*p.

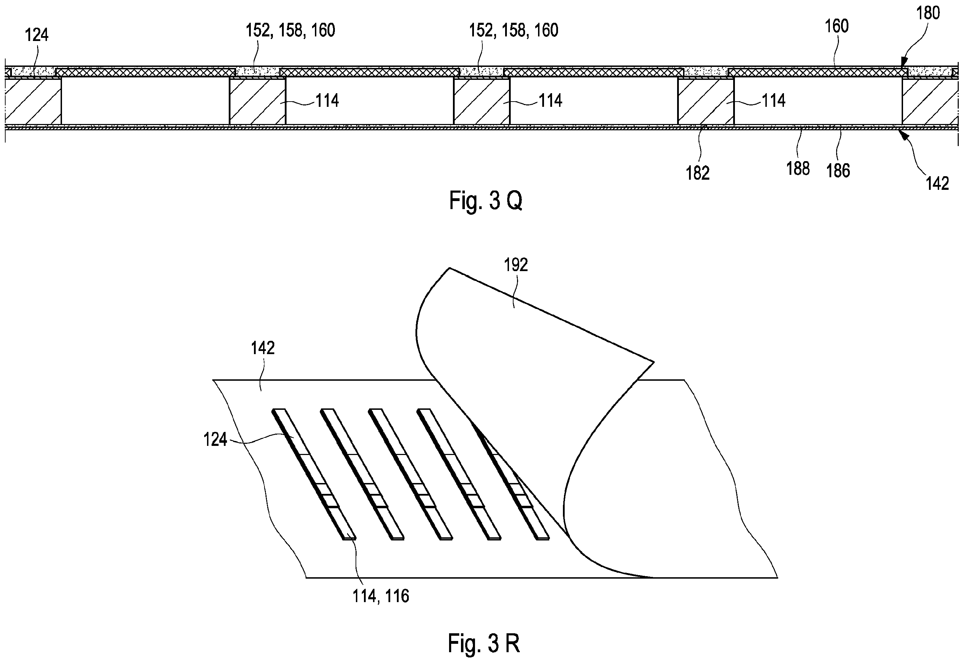

The exemplary embodiment the support foil 142 having the core strips 114 transferred thereto is shown in Figure 3Q in a schematic side cut view along the third line 158 of through holes 152. As can be seen in Figure 3Q, the contact sides 182 of the core strips 114 may face towards the support foil 142 and the analytical elements 124 may face away from the support foil 142, specifically being covered by the inversion foil 180.

Further, the method may comprise cutting the third lines 158 of through holes 152, removing the transfer foil 128 and/or the inversion foil 180 and covering the core strips 114 on the support foil 142 with at least one cover sealing foil 192 (denoted by reference number 194). The covering of the core strips 114 on the support foil 142 is exemplarily shown in Figure 3R in a top view. Figure 3S shows the covered core strips 114 in a schematic side cut view. Additionally, the covering of core strips 114 on the support foil 142 with the cover sealing foil 192 may comprise sealing the core strips 114. In this example, the cover sealing foil 192 may be an aluminum foil. The sealing may comprise welding the cover sealing 192 to the support foil 142, more specifically welding in between and/or around the core strips 114 on the support foil 142. The sealing may specifically be a hermetic sealing of the core strips 114. Thus, by sealing the core strips 114, the core strips 114 comprised by the analytical test element 112 may be enclosed in an air- and watertight environment in between the support foil 142 and the cover sealing foil 192.

Further, the method may comprise applying perforation lines 196 into the support foil 142 with the core strips 114 disposed thereon, and also into the cover sealing foil 192, such that at least one perforation line 196 is disposed in between neighboring core strips 114. The manufactured plurality of analytical test elements 112 is shown in Figure 3T in a top view and in Figure 3U in a bottom view. As can be seen in Figures 3T and 3U, the plurality of analytical test elements 112 produced by the manufacturing method according to the present invention may be identified by being assembled in units of manufactured plurality of analytical test elements 112, e.g. being assembled in roll ware units or sheet ware units of 10, 20, 50, or even 100 analytical test elements 112 having the perforation lines 196 in between neighboring analytical test elements 112. The plurality of analytical test elements 112, as can be seen in Figures 3T and 3U, each may comprise an indication for opening the analytical test element 112, such as an arrow indicating an opening direction, specifically for simple opening of the analytical test element 112, e.g. by separating the cover seal foil 192 from the support foil 142 thereby revealing the core strips 114 with the analytical element 124 comprised by the analytical test element 112.

List of reference numbers

[0110]

| | |
|---|---|
| 110 | manufacturing device |
| 112 | analytical test element |
| 114 | core strip |
| 116 | support foil strip |
| 118 | core strip intermediate product supply device |
| 120 | core strip intermediate product |
| 122 | carrier foil |
| 124 | analytical element |
| 126 | transfer foil application device |
| 128 | transfer foil |
| 130 | front side |
| 132 | reverse side |
| 134 | first transfer intermediate product |
| 136 | second transfer intermediate product |
| 138 | separation device |
| 140 | transfer device |
| 142 | support foil |
| 144 | providing the core strip intermediate product |
| 145 | pitch p |
| 146 | cutting lines |
| 148 | rim portions |
| 150 | applying transfer foils |
| 152 | through holes |
| 154 | first line of through holes |
| 156 | second line of through holes |
| 158 | third line of through holes |
| 160 | adhesive tape |
| 162 | positioning marks |
| 164 | separating the first and second transfer intermediate products |
| 166 | further transfer foil |
| 168 | applying the further transfer foil |
| 170 | third transfer intermediate product |
| 172 | removing the further transfer foil |
| 174 | fourth transfer intermediate product |
| 176 | fifth transfer intermediate product |
| 178 | inverting the core strips |
| 180 | inversion transfer foil |
| 182 | contact sides |
| 184 | transferring the core strips onto the support foil |
| 186 | aluminum foil |
| 188 | adhesive foil |
| 190 | separation distance |
| 192 | cover sealing foil |
| 194 | covering the core strips on the support foil |
| 196 | perforation line |

## Claims

1. A method of manufacturing a plurality of analytical test elements (112) comprising at least one core strip (114) and at least one support foil strip (116), the method comprising:

   i. providing at least one core strip intermediate product (120), the core strip intermediate product (120) comprising at least one carrier foil (122) and at least one analytical element (124) attached to the at least one carrier foil (122), the carrier foil (122) and the attached analytical element (124), in the carrier intermediate product (120), being cut such that a plurality of core strips (114) of the analytical test elements (112) is defined, the core strips (114) being placed adjacent to each other;

   ii. applying transfer foils (128) on a front side (130) and on a reverse side (132) of the core strip intermediate product (120), wherein the transfer foil (128) applied to the front side (130) and the transfer foil (128) applied to the reverse side (132) engage with the core strips (114) in an alternating fashion, the transfer foil (128) on the front side (130) and the core strips (114) engaging with the transfer foil (128) on the front side (130) forming a first transfer intermediate product (134), the transfer foil (128) on the reverse side (132) and the core strips (114) engaging with the transfer foil (128) on the reverse side (132) forming a second transfer intermediate product (136);

   iii. separating the first (134) and second transfer intermediate products (136); and

   iv. transferring the core strips (114) onto at least one support foil (142), wherein the core strips (114), on the support foil (142), are spaced apart from each other by at least one width of one core strip (114).

2. The method according to the preceding claim, wherein the method is performed as a reel-to-reel process.

3. The method according to any one of the preceding claims, the method further comprising at least one cutting process, wherein, in the cutting process, at least one part of at least one element is cut, wherein the at least one element is selected from the group consisting of: the core strip intermediate product (120); the first transfer intermediate product (134); the second transfer intermediate product (136).

4. The method according to any one of the preceding claims, further comprising:

   v. applying at least one further transfer foil (166) on at least one of the first (134) and second transfer intermediate products (136), such that the core strips (114) are embedded in between the transfer foils (128, 166), wherein the further transfer foil (166) engages with every second of the core strips (114) located on the at least one of the first (134) and second transfer intermediate products (136), thereby generating a third transfer intermediate product (170); and

vi. removing the further transfer foil (166) with the core strips (114) engaging therewith from the third transfer intermediate product (170), such that the further transfer foil (166) with the core strips (114) engaging therewith form a fourth transfer intermediate product (174) and that the remaining transfer foil (128) of the third transfer intermediate product (170) and the core strips (114) engaging therewith after the removal of the further transfer foil (166) form a fifth transfer intermediate product (176).

**5.** The method according to any one of the preceding claims, wherein the transfer foils (128) comprise a plurality of through holes (152) arranged in lines, wherein the method further comprises applying at least one adhesive tape (160) to the transfer foils (128) in such a way that at least one adhesive region of the adhesive tape (160) engages with the carrier foil (122) of the core strips (114) through the through holes (152).

**6.** The method according to the preceding claim, wherein the core strips (114) being placed adjacent to each other on the core strip intermediate product (120) have a pitch p (145), wherein the through holes (152) of each line have a pitch s, with:

$$s = 2^m * p,$$

with m being a positive integer.

**7.** The method according to the preceding claim, wherein the transfer foils (128) each comprise a plurality of at least two different lines of through holes (152), the at least two different lines of through holes (152) differing from each other with respect to the pitch s of their through holes (152).

**8.** The method according to any one of the two preceding claims, wherein, in step ii., the transfer foils (128) on the front side (130) and on the reverse side (132) are offset in a longitudinal direction of the transfer foils (128) by an offset distance of s/2, such that the transfer foils (128) on opposing sides of the core strips (114), via their respective adhesive tapes (160), engage with the core strips (114) in an alternating fashion.

**9.** The method according to any one of the four preceding claims, the method further comprising at least one edge cutting process, wherein, in the edge cutting process, at least one part of at least one of the transfer foils (128) is cut off, wherein the at least one part being cut off contains at least one line of through holes (152).

**10.** The method according to any one of the preceding claims, wherein the core strip intermediate (120) product further comprises rim portions (148), wherein the plurality of core strips (114) is located in between the rim portions (148), the method further comprising removing the rim portions (148), before separating the first (134) and second transfer intermediate product (136) in step iii..

**11.** The method according to any one of the preceding claims, further comprising:
vii. covering the core strips (114) on the support foil (142) with at least one cover sealing foil.

**12.** The method according to any one of the preceding claims, wherein the core strips (114) being placed adjacent to each other on the core strip intermediate product (120) have a pitch p (145), wherein, on the support foil (142), the core strips (114) are spaced apart from each other by a separation distance d (190):

$$d = (2^n - 1)*p,$$

with n being a positive integer.

**13.** The method according to any one of the preceding claims, wherein the core strips (114) each have a contact side (182) and, in step iv., are applied to the support foil (142) with their contact sides (182), wherein some of the transfer intermediate products (134, 136, 170, 174, 176) are embodied such that the contact sides (182) of the core strips (114) faced towards the transfer foils (128) of the respective transfer intermediate products (134, 136, 170, 174, 176), wherein the method further comprises:
viii. inverting the core strips (114) having contact sides (182) facing towards their respective transfer foil (128), the inverting comprising applying an inversion transfer foil (180) onto the core strips (114) on a side opposing the contact sides (182) of the core strips (114), the inverting further comprising removing the transfer foil (128) from the contact sides (182) of the core strips (114).

**14.** A manufacturing device (110) for manufacturing a plurality of analytical test elements (112) comprising at least one core strip (114) and at least a support foil strip (116), wherein the manufacturing device (100) comprises:

I. at least one core strip intermediate product supply device (118) configured for supplying at least one core strip intermediate product (120), the core strip intermediate product (120) comprising at least one carrier foil (122) and at least one analytical element (124) attached to the at least one carrier foil (122), the carrier foil (122) and the attached analytical element (124), in the core strip intermediate product (120), being cut such that a plurality of core strips (114) of the analytical test elements (112) is defined, the core strips (114) being placed adjacent to each other;

II. at least one transfer foil application device (126) configured for applying transfer foils (128) on a front side (130) and on a reverse side (132) of the core strip intermediate product (120), wherein the transfer foil (128) applied to the front side (130) and the transfer foil (128) applied to the reverse side (132) engage with the core strips (114) in an alternating fashion, the transfer foil (128) on the front side (130) and the core strips (114) engaging with the transfer foil (128) on the front side (130) forming a first transfer intermediate product (134), the transfer foil (128) on the reverse side (132) and the core strips (114) engaging with the transfer foil (128) on the reverse side (132) forming a second transfer intermediate product (136);

III. at least one separation device (138) configured for separating the first (134) and second transfer intermediate products (136); and

IV. at least one transfer device (140) configured for transferring the core strips (114) onto at least one support foil (142), such that the core strips (114), on the support foil (142), are spaced apart from each other by at least one width of one core strip (114).

15. A plurality of analytical test elements (112), the plurality of analytical test elements (112) being produced by the method according to any one of the preceding claims referring to a method of manufacturing a plurality of analytical test elements (112).

Fig. 1

Fig. 2

Fig. 3 A

Fig. 3 B

EP 4 462 122 A1

Fig. 3 C

Fig. 3 D

Fig. 3 E

EP 4 462 122 A1

Fig. 3 F

Fig. 3 G

Fig. 3 H

Fig. 3 I

Fig. 3 J

Fig. 3 K

EP 4 462 122 A1

Fig. 3 L

Fig. 3 M

Fig. 3 N

EP 4 462 122 A1

Fig. 3 P

Fig. 3 O

Fig. 3 Q

Fig. 3 R

EP 4 462 122 A1

Fig. 3 S

Fig. 3 T

Fig. 3 U

EP 4 462 122 A1

Europäisches
Patentamt

European
Patent Office

Office européen
des brevets

# EUROPEAN SEARCH REPORT

Application Number

EP 23 17 2075

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 4 151 151 A1 (HOFFMANN LA ROCHE [CH]; ROCHE DIABETES CARE GMBH [DE]) 22 March 2023 (2023-03-22) * paragraph [0025] * * paragraph [0189] * * paragraph [0204] * * claim 14 * | 1-15 | INV. G01N33/543 B01L3/00 ADD. G01N35/00 B32B37/00 |
| A | US 8 828 912 B2 (COONEY CHRISTOPHER G [US]; AKONNI BIOSYSTEMS INC [US]) 9 September 2014 (2014-09-09) * column 10, lines 17-36 * * column 12, lines 23-33 * * column 13, line 14 – column 14, line 12 * * figure 5 * | 1-15 | |
| A | US 8 608 891 B2 (AYLIFFE HAROLD E [US]; KING CURTIS S [US]; EI SPECTRA LLC [US]) 17 December 2013 (2013-12-17) * claim 11 * * figure 17 * | 1-15 | |

TECHNICAL FIELDS
SEARCHED (IPC)

G01N
B01L
B32B
A61B

The present search report has been drawn up for all claims

| Place of search | Date of completion of the search | Examiner |
|---|---|---|
| The Hague | 28 September 2023 | Traversa, Marzia |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

    .....................................................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 23 17 2075

This annex lists the patent family members relating to the patent documents cited in the above-mentioned European search report.
The members are as contained in the European Patent Office EDP file on
The European Patent Office is in no way liable for these particulars which are merely given for the purpose of information.

28-09-2023

| Patent document cited in search report | | | Publication date | Patent family member(s) | | | Publication date |
|---|---|---|---|---|---|---|---|
| EP 4151151 | A1 | | 22-03-2023 | EP | 4151151 | A1 | 22-03-2023 |
| | | | | TW | 202321686 | A | 01-06-2023 |
| | | | | WO | 2023046606 | A1 | 30-03-2023 |
| US 8828912 | B2 | | 09-09-2014 | CA | 2870069 | A1 | 18-10-2012 |
| | | | | CA | 3119914 | A1 | 18-10-2012 |
| | | | | CN | 103635802 | A | 12-03-2014 |
| | | | | CN | 105289762 | A | 03-02-2016 |
| | | | | CN | 107469878 | A | 15-12-2017 |
| | | | | EP | 2697648 | A2 | 19-02-2014 |
| | | | | EP | 3236260 | A1 | 25-10-2017 |
| | | | | EP | 3425396 | A2 | 09-01-2019 |
| | | | | ES | 2698901 | T3 | 06-02-2019 |
| | | | | HK | 1195810 | A1 | 21-11-2014 |
| | | | | HK | 1220944 | A1 | 19-05-2017 |
| | | | | HK | 1249475 | A1 | 02-11-2018 |
| | | | | US | 2012196767 | A1 | 02-08-2012 |
| | | | | US | 2014287954 | A1 | 25-09-2014 |
| | | | | US | 2017016052 | A1 | 19-01-2017 |
| | | | | US | 2020002755 | A1 | 02-01-2020 |
| | | | | US | 2021215689 | A1 | 15-07-2021 |
| | | | | WO | 2012142397 | A2 | 18-10-2012 |
| US 8608891 | B2 | | 17-12-2013 | CN | 101999071 | A | 30-03-2011 |
| | | | | EP | 2265923 | A1 | 29-12-2010 |
| | | | | JP | 5303028 | B2 | 02-10-2013 |
| | | | | JP | 2011516884 | A | 26-05-2011 |
| | | | | US | 2011030888 | A1 | 10-02-2011 |
| | | | | US | 2012261067 | A1 | 18-10-2012 |
| | | | | WO | 2009126257 | A1 | 15-10-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20220305766 A1
- US 20020076828 A1
- US 6207000 B1
- US 20080289749 A1
- EP 2907573 A1
- US 20050118662 A1